# EUROPEAN PATENT SPECIFICATION

(11) **EP 4 379 062 B1**
(45) Date of publication and mention of the grant of the patent: **11.11.2026**
(21) Application number: 22383159.5
(22) Date of filing: 30.11.2022
(51) Int. Cl.: C12Q 1/26

(54) **BIOSYSTEM FOR PESTICIDE DETECTION**
BIOSYSTEM ZUM NACHWEIS VON PESTIZIDEN
BIOSYSTÈME POUR LA DÉTECTION DE PESTICIDES

(43) Date of publication of application: 05.06.2024
(73) Proprietor: Fundación AZTI - AZTI Fundazioa, 48160 Derio (Bizkaia) (ES)
(72) Inventor: Barranco, Alejandro, BIZKAIA (ES); Cunha, Hugo, BIZKAIA (ES); Ereño-Artabe, Amaia, BIZKAIA (ES)
(74) Representative: Pons IP

(56) References cited:
- WO-A1-2022/144095
- CN-A- 108 414 639
- US-A1- 2004 014 772
- BUCUR BOGDAN ET AL: "Advances in Enzyme-Based Biosensors for Pesticide Detection", BIOSENSORS, vol. 8, no. 2, 22 March 2018 (2018-03-22), pages 27, XP093044684, DOI: 10.3390/bios8020027
- XU YU-LING ET AL: "Cholinesterases and Engineered Mutants for the Detection of Organophosphorus Pesticide Residues", SENSORS, vol. 18, no. 12, 5 December 2018 (2018-12-05), pages 4281, XP093044682, DOI: 10.3390/s18124281

## Description

The invention belongs to the field of food and feed safety, human, animal and plant health sector and environmental control. Particularly, the present invention describes a method for the detection of pesticides, and a kit and uses for that purpose, based on particular cytochrome CYP450 enzymes, CYP2B11 and CYP2B10.

### BACKGROUND ART

Pesticides are one of the main groups of xenobiotics present in the environment, having an important impact, especially, in agriculture areas. Furthermore, since pesticides are widely used in agriculture, related quality and safety challenges associated to food and feed industry arise.

Therefore, it is of great importance have detection methods for pesticide screening. Traditional pesticide detection technologies mainly include gas chromatography, high performance liquid chromatography (HPLC) and their combination with mass spectrometry. Approaches like these are described in the state of the art.

CN109298111A describes a method for pesticide simultaneous detection in fruits and vegetables, using HPLC-MS (High Performance Liquid Chromatography-Mass Spectrometry) as machine detection method. The content of each pesticide residue in the detected sample can be determined through sample processing, standard curve establishment and final calculation according to standard curve.

CN108414639A discloses a method for pesticide detection in food, which includes processing food step and performing GC-MS (Gas chromatography-mass spectrometry) quantitative analysis on the preprocessed sample to obtain the content of the pesticide residues in the food.

However, the above methods comprise the use of instrumentation and equipment that are cumbersome and expensive. Recently, approaches based on enzymatic inhibition is gaining importance.

CN101832986A discloses a kit for detecting pesticide using a thin layer chromatography-enzymatic inhibition method, comprising the kit a cured cholinesterase bar, a thin layer chromatography board, a developing agent, a substrate developing bar and standard pesticide, wherein the cured cholinesterase bar comprises a strip vector and cholinesterase cured on the strip carrier.

In Badawy SM. Validation and kinetic of enzymatic method for the detection of organophosphate insecticides based on cholinesterase inhibition. Toxicol Mech Methods. 2020 Feb;30(2):134-138 scientific article, kinetic and validation of the enzymatic method for the determination of fenitrothion organophosphorus pesticide based on cholinesterase inhibition were studied.

WO2022/144095 discloses an enzyme inhibition assay using CYP450 enzymes for detecting chemical contaminants in a sample. This document however does not disclose the detection of pesticides.

As pesticides are used extensively, creating serious acute health problems and local and global environmental contaminations, there is a need in the state of the art of providing alternative approaches for fast and efficient pesticide detection, including simultaneous multiple pesticide detection.

### DESCRIPTION OF THE INVENTION

The scope of the invention is defined in the appended claims. Embodiments and descriptions not falling under the scope of the appended claims are considered merely as examples suitable for understanding the invention.

Inventors have developed an approach related to pesticide detection based on the inhibition generated by these substances on the enzymatic activity of CYP2B11 and/or CYP2B10, which are enzymes belonging to the CYP450 superfamily. Inventors have demonstrated that enzyme CYP2B11 and CYP2B10 are inhibited by a wide range of pesticide molecules, as shown in Table 7, which is used as a basis for pesticide detection. In addition, an optimization of an extraction protocol related to the pesticide detection in mushrooms were performed. The optimized protocol allows the obtention of fungi extracts compatible with the enzymatic assay-based pesticide detection and with the required sensitivity (Example 7). Furthermore, as shown in Table 6, it was validated the use of CYP2B10 enzyme to determine prochloraz and chlorpyrifos-methyl in mushrooms, at 0.01 mg/kg, demonstrating that the approach is of relevance, since for instance, the EU regulation establishes the maximum residues levels (MRLs) of 3 mg/kg for prochloraz (Reg. (EU) 2020/192) and 0.01 mg/kg for chlorpyrifos-methyl (Reg. (EU) 2020/1085) in cultivated fungi.

Thus, the invention provides an alternative approach compared to the methods known in the state of the art, having several advantages: the use of mentioned CYP450 enzymes allows simultaneous pesticide detection being able to detect the presence of multiple pesticides belonging to different chemical families; detection is very fast, being able to achieve pesticide detection in less than 1 hour; it does not require complex or expensive equipment and it is easily implementable in industrial installations; the method is compatible with different signal detection techniques; the enzymes used in the present invention, CYP2B11 and CYP2B10, show, for instance, selective inhibition to some pesticide molecules not observed with the human homologue (CYP2B6).

Thus, the present invention describes a method for pesticide detection and kits and uses for such purpose, which will be described in detailed below.

### Method of the invention

Based on the inhibition produced by pesticides in specific cytochromes P450 (CYP450) enzymes, particularly, CYP2B11 and/or CYP2B10, the inventors have developed a method for pesticide detection in a sample.

Thus, an aspect of the invention relates to a method for pesticide detection in a sample, hereinafter the "method of the invention", comprising the following steps:
a) contacting the sample or an extract thereof, with at least one CYP450 enzyme selected from the list consisting of CYP2B11 enzyme and CYP2B10 enzyme,
b) addition of, at least, one enzyme reporter substrate,
c) detection and measurement of a detectable signal, and
d) determination of pesticide presence in the sample by comparing the measured detectable signal with a signal from a reference sample.

The term "pesticide", as used herein, refers to any substance used to kill, repel, or control pests. Pesticides can include a wide spectrum of chemicals, such as insecticides, rodenticides, herbicides, fungicides, biocides, and related chemicals.

Examples of pesticides include, without limitation to, benalaxyl, captan, chlorpyrifos, chlorpyrifos methyl, chlorothalonil, cyazofamid, cypermethrin, DDT, deltamethrin, diazinon, dicofol, dimethoate, epoxiconazole, fenbuconazole, fenitrothion, folpet, mancozeb, metrafenone, meptyldinocap, myclobutanil, penconazole, phosmet, piperonyl butoxide, pirimiphos methyl, prochloraz, procymidone, propiconazole, or simazine.

In the present invention, the term "detection", as used herein, refers to the identification of the presence of one or more pesticides in a sample. Multianalyte detection, in the present invention the simultaneous detection of multiple pesticides, is also considered.

In a preferred embodiment of the invention, alone or in combination with the rest of preferred embodiments pesticide detection comprises the detection of 2, 3, 4, 5, 6, 7, 8, 9, 10, 11, 12, 13, 14, 15 or more pesticides.

Another preferred embodiment of the method of the invention, alone or in combination with the rest of preferred embodiments, provides the method of the invention wherein the pesticide detection comprises the detection of at least one pesticide, wherein the pesticide belongs to a family of pesticides selected from the list consisting of amide, aryl phenyl ketone, azole, benzophenone, dicarboximide, dinitrophenol, dithiocarbamate, organobromide, organochlorine, organophosphate, phthalimide, pyrethroid, triazine, synergists molecules and any combination thereof. In the method of the invention, when the pesticide detection comprises the detection of 2, 3, 4, 5, 6, 7, 8, 9, 10, 11, 12, 13, 14, 15 or more pesticides, the pesticides can be from any of the families above mentioned, and any combination thereof.

In a more preferred embodiment of the method of the invention, when the pesticide belongs to the amide family, the pesticide is benalaxyl.

In another more preferred embodiment of the method of the invention, when the pesticide belongs to the aryl phenyl ketone, benzophenone or organobromide family, the pesticide is metrafenone.

In another more preferred embodiment of the method of the invention, when the pesticide belongs to the azole family, the pesticide is selected from the list consisting of cyazofamid, epoxiconazole, fenbuconazole, myclobutanil, penconazole, prochloraz, propiconazole, and any combination thereof.

In another more preferred embodiment of the method of the invention, when the pesticide belongs to the dicarboximide family, the pesticide is procymidone.

In another more preferred embodiment of the method of the invention, when the pesticide belongs to the dinitrophenol family, the pesticide is meptyldinocap.

In another more preferred embodiment of the method of the invention, when the pesticide belongs to the dithiocarbamate family, the pesticide is mancozeb.

In another more preferred embodiment of the method of the invention, when the pesticide belongs to the organochlorine family, the pesticide is selected from the list consisting of chlorothalonil, DDT, dicofol, and any combination thereof.

In another more preferred embodiment of the method of the invention, when the pesticide belongs to the organophosphate family, the pesticide is selected from the list consisting of chlorpyrifos, chlorpyrifos methyl, diazinon, dimethoate, fenitrothion, pirimiphos methyl, and any combination thereof.

In another more preferred embodiment of the method of the invention, when the pesticide belongs to the phthalimide family, the pesticide is selected from the list consisting of captan, folpet, and its combination.

In another more preferred embodiment of the method of the invention, when the pesticide belongs to the phthalimide or organophosphate family, the pesticide is phosmet.

In another more preferred embodiment of the method of the invention, when the pesticide belongs to the pyrethroid family, the pesticide is cypermethrin, deltamethrin, and its combination.

In another more preferred embodiment of the method of the invention, when the pesticide belongs to the triazine family, the pesticide is simazine.

In another more preferred embodiment of the method of the invention, when the pesticide belongs to the synergist family, the pesticide is piperonyl butoxide.

In another embodiment of the method of the invention, alone or in combination with the rest of preferred embodiments, provides the method of the invention wherein the pesticide detection comprises the detection of at least one pesticide selected from the list consisting of benalaxyl, captan, chlorpyrifos, chlorpyrifos methyl, chlorothalonil, cyazofamid, cypermethrin, DDT, deltamethrin, diazinon, dicofol, dimethoate, epoxiconazole, fenbuconazole, fenitrothion, folpet, mancozeb, meptyldinocap, metrafenone, myclobutanil, penconazole, phosmet, piperonyl butoxide, pirimiphos methyl, prochloraz, procymidone, propiconazole, simazine and any combination thereof.

In a more preferred embodiment, alone or in combination with the rest of preferred embodiments, pesticide detection comprises the detection of prochloraz and/or chlorpyrifos-methyl.

### Step (a) of the method of the invention

In a first step [step (a)], the method of the invention comprises contacting the sample or an extract thereof, with at least one CYP450 enzyme selected from the list consisting of CYP2B11 enzyme and CYP2B10 enzyme.

CYP2B11 and CYP2B10 are kinds of enzymes belonging to the superfamily of CYP450 enzymes. In particular, CYP2B11 which can also be referred in the present document as canine or dog CYP2B11, and CYP2B10, which can also be referred in the present document as mouse CYP2B10, are members of CYP2B subfamily (whose homologue in humans is CYP2B6), being important in the metabolism and detoxification of clinical drugs and other exogenous substances, as well as other endogenous compounds.

In a preferred embodiment, alone or in combination with the rest of preferred embodiments, CYP2B11 comprises, or consists of, an amino acid sequence with a sequence identity of, at least, 70, 75, 80, 85, 90, 91, 92, 93, 94, 95, 96, 97, 98 or 99% to SEQ ID NO: 1.

SEQ ID NO: 1
MELSVLLLLALLTGLLLLMARGHPKAYGHLPPGPRPLPILGNFLQMDRKGLLKSFLR
LQEKYGDVFTVYLGPRRTVMLCGIDAIREALVDNAEAFSGRGKIAVVEPVFQGYGV
VFANGERWKTLRRFSLATMRDFGMGKRSVEERIQEEAQCLVEELRKTEGVLQDPT
FFFHSMTANIICSIVFGKRFGYKDPEFLRLMNLFYVSFALISSFSSQMFELFHSFLKY
FPGTHRQVYNNLQEIKAFIARMVEKHRETLDPSAPRDFIDAYLIRMDKEKAEPSSEF
HHRNLIDTALSLFFAGTETTSTTLRYGFLLMLKYPHIAERIYKEIDQVIGPHRLPSLDD
RAKMPYTDAVIHEIQRFGDLLPIGVPHMVTKDICFRGYIIPKGTEVFPILHSALNDPH
YFEKPDVFNPDHFLDANGALKKNEAFIPFSIGKRICLGEGIARMELFLFFTTILQNFS
VASPMAPEDIDLTPQEIGVGKLPPVYQISFLSRGGC

SEQ ID NO: 1 corresponds to CYP2B11 amino acid sequence (NCBI Reference Sequence: NP_001006653.1; organism: *Canis lupus familiaris* (dog)). In a more preferred embodiment of the method of the invention, alone or in combination with the rest of preferred embodiments, the CYP2B11 comprises, or consists of, SEQ ID NO: 1.

In a preferred embodiment, alone or in combination with the rest of preferred embodiments, CYP2B10 comprises, or consists of, an amino acid sequence with a sequence identity of, at least, 70, 75, 80, 85, 90, 91, 92, 93, 94, 95, 96, 97, 98 or 99% to SEQ ID NO: 2.

SEQ ID NO: 2
MEPSVLLLLALLVGFLLLLARGHPKSRGNFPPGPRPLPLLGNLLQMDRGGLLKSFIQ
LREKYGDVFTVHLGPRPVVMLCGTDTIREALVGQAEAFSGRGTVAVVEPTFKEYG
VIFANGERWKTLRRFSLATMRDFGMGKRSVEERIQEEAQCLVEELRKSQGAPLDP
TFLFQCITANIICSIVFGERFEYTDRQFLRLLELFYQTFSLISSFSSQMFELFSGFLKY
FPGAHRQISKNLQELLDYIGHSVEKHRATLDPSVPRDFIDIYLLRMEKEKSNQHTEF

HHQNLMMSVLSLFFAGTETSSTTLRYGFLLMLKYPHVAEKVQKEIDQVIGSHRLPT
LDDRTKMPYTDAVIHEIQRFSDLIPIGVPHRVTKDTMFRGYLLPKNTEVYPILSSALH
DPQYFEQPDSFNPDHFLDANGALKKSEAFLPFSTGKRICLGESIARNELFLFFTSIL
QNFSVASHVAPKDIDLTPKESGIGKIPPTYQICFLAR

SEQ ID NO: 2 corresponds to CYP2B10 amino acid sequence (NCBI Reference Sequence: NP_034129.1; organism: *Mus musculus* (mouse)). In a more preferred embodiment of the method of the invention, alone or in combination with the rest of preferred embodiments, the CYP2B10 comprises, or consists of, SEQ ID NO: 2.

In the present invention, "sequence identity" means the degree of similarity between two amino acid sequences obtained by aligning the two sequences. Depending on the number of common residues between the aligned sequences, a degree of identity expressed as a percentage will be obtained. The degree of identity between two amino acid sequences can be determined by conventional methods, e.g. by standard sequence alignment algorithms known in the state of the art, such as BLAST [Altschul S.F. et al. Basic local alignment search tool. J Mol Biol. 1990 Oct 5; 215(3):403-10]. BLAST programs, e.g. BLASTN, BLASTX, and TBLASTX, BLASTP and TBLASTN, are in the public domain on the website of The National Center for Biotechonology Information (NCBI).

The term "contacting" as used in the present invention, refers to put in contact the sample or an extract thereof with, at least, one CYP450 enzyme selected from the list consisting of CYP2B11 enzyme and CYP2B10 enzyme, allowing the interaction between the pesticide/s present in the sample and the CYP450 enzyme/s.

In a preferred embodiment of the method of the invention, alone or in combination with the rest of preferred embodiments, when the step (a) comprises contacting the sample or an extract thereof, with a CYP2B11 enzyme, the pesticide detection comprises the detection of at least one pesticide belonging to the pesticide family selected from the list consisting of amide, aryl phenyl ketone, azole, benzophenone, dicarboximide, dinitrophenol, dithiocarbamate, organobromide, organochlorine, organophosphate, phthalimide, phthalimide/organophosphate, pyrethroid, triazine, synergists molecules and any combination thereof. More preferably, when the step (a) comprises contacting the sample or an extract thereof, with a CYP2B11 enzyme, the pesticide detection comprises the detection of at least one pesticide selected from the list consisting of benalaxyl, captan, chlorpyrifos, chlorpyrifos methyl, chlorothalonil, cyazofamid, cypermethrin, DDT, deltamethrin, diazinon, dicofol, dimethoate, epoxiconazole, fenbuconazole, fenitrothion, folpet, mancozeb, meptyldinocap, metrafenone, myclobutanil, penconazole, phosmet, piperonyl butoxide, pirimiphos methyl, prochloraz, procymidone, propiconazole, simazine and any combination thereof.

In a preferred embodiment of the method of the invention, alone or in combination with the rest of preferred embodiments, when the step (a) comprises contacting the sample or an extract thereof, with a CYP2B10 enzyme, the pesticide detection comprises the detection of at least one pesticide belonging to the pesticide family selected from the list consisting of azole, organochlorine, organophosphate, phthalimide, phthalimide/organophosphate, triazine, and any combination thereof. More preferably, when the step (a) comprises contacting the sample or an extract thereof, with a CYP2B10 enzyme, the pesticide detection comprises the detection of at least one pesticide selected from the list consisting of captan, chlorpyrifos, chlorpyrifos methyl, diazinon, dicofol, fenbuconazole, fenitrothion, folpet, myclobutanil, penconazole, phosmet, pirimiphos methyl, prochloraz, propiconazole, simazine and any combination thereof.

In a preferred embodiment of the method of the invention, alone or in combination with the rest of preferred embodiments, the step (a) comprises contacting the sample or an extract thereof, with a CYP2B11 enzyme and a CYP2B10 enzyme. In a more preferred embodiment of the method of the invention, when the step (a) comprises contacting the sample or an extract thereof, with a CYP2B11 enzyme and a CYP2B10 enzyme, the pesticide detection comprises the detection of, at least, one pesticide belonging to the pesticide family selected from the list consisting of amide, aryl phenyl ketone, azole, benzophenone, dicarboximide, dinitrophenol, dithiocarbamate, organobromide, organochlorine, organophosphate, phthalimide, pyrethroid, triazine, synergists molecules and any combination thereof. More preferably, when the step (a) comprises contacting the sample or an extract thereof, with a CYP2B10 enzyme and a CYP2B11 enzyme, the pesticide detection comprises the detection of at least one pesticide selected from the list consisting of benalaxyl, captan, chlorpyrifos, chlorpyrifos methyl, chlorothalonil, cyazofamid, cypermethrin, DDT, deltamethrin, diazinon, dicofol, dimethoate, epoxiconazole, fenbuconazole, fenitrothion, folpet, mancozeb, meptyldinocap, metrafenone, myclobutanil, penconazole, phosmet, piperonyl butoxide, pirimiphos methyl, prochloraz, procymidone, propiconazole, simazine and any combination thereof.

In another preferred embodiment, alone or in combination with the rest of preferred embodiments, when the sample is a mushroom sample or a mushroom extract, the pesticide detection comprises the detection of, at least, one pesticide selected from the list consisting of prochloraz, procymidone, chlorothalonil, dicofol, chlorpyrifos, chlorpyrifos-methyl, diazinon, piperonyl butoxide, cypermethrin, metrafenone and/or deltamethrin.

In another preferred embodiment, alone or in combination with the rest of preferred embodiments, the step (a) of the method of the invention is carried out during less than 10 min.

In another preferred embodiment, alone or in combination with the rest of preferred embodiments, the step (a) of the method of the invention is carried out at a temperature of 20 ºC to 40 ºC (including the end values of the range), preferably at a temperature selected from the list consisting of 20, 21, 22, 23, 24, 25, 26, 27, 28, 29, 30, 31, 32, 33, 34, 35, 36, 37, 38, 39, and 40ºC.

In a more preferred embodiment of the method of the invention, alone or in combination with the rest of preferred embodiments, the step (a) of the method of the invention is carried out during less than 10 min at a temperature of 20 ºC to 40 ºC (including the end values of the range), preferably at a temperature selected from the list consisting of 20, 21, 22, 23, 24, 25, 26, 27, 28, 29, 30, 31, 32, 33, 34, 35, 36, 37, 38, 39, and 40ºC.

In another preferred embodiment, alone or in combination with the rest of preferred embodiments, the step (a) is carried out at a pH between 6 to 9 (including the end values of the range). More preferably, the step (a) is carried out at a pH selected from the list consisting of 6, 7, 8, and 9.

Furthermore, the CYP2B11 enzyme and/or the CYP2B10 enzyme can be contacted with the sample or an extract thereof as part of an enzymatic mixture in presence of buffers and/or solvents known in the state of the art.

In a preferred embodiment, alone or in combination with the rest of the preferred embodiments, the step (a) is carried out in presence of a buffer which allows to reach a pH between 6 to 9. More preferably, the step (a) is carried out in presence of a buffer selected from the list consisting of phosphate buffer, Tris-based buffers, HEPES (4-(2-hydroxyethyl)-1-piperazineethanesulfonic acid) and PIPES (piperazine-1,4-bis(2-ethanesulfonic acid). More preferably, the step (a) is carried out in presence of potassium phosphate buffer, wherein potassium phosphate buffer is at a concentration between 10 mM to 200 mM (including the end values of the range).

In a still more preferred embodiment, the step (a) is carried out during less than 10 min at a temperature of 20 ºC to 40 ºC (including the end values of the range), preferably at a temperature selected from the list consisting of 20, 21, 22, 23, 24, 25, 26, 27, 28, 29, 30, 31, 32, 33, 34, 35, 36, 37, 38, 39, and 40ºC; and in presence of a buffer which allows to reach a pH between 6 to 9, preferably wherein the buffer is selected from the list consisting of phosphate buffer, Tris-based buffers, HEPES (4-(2-hydroxyethyl)-1-piperazineethanesulfonic acid) and PIPES (piperazine-1,4-bis(2-ethanesulfonic acid), more preferably wherein the buffer is at a concentration between 10 mM to 200 mM (including the end values of the range).

In another preferred embodiment, alone or in combination with the rest of the preferred embodiments, the step (a) is carried out in presence of a solvent selected from the list consisting of acetonitrile, acetone, dimethyl sulfoxide (DMSO), acetone, dimethylformamide (DMF), ethanol and methanol. More preferably, the step (a) is carried out in presence of an acetonitrile solvent, wherein acetonitrile concentration is below 15%.

In a still more preferred embodiment, the step (a) is carried out during less than 10 min at a temperature of 20 ºC to 40 ºC (including the end values of the range), preferably at a temperature selected from the list consisting of 20, 21, 22, 23, 24, 25, 26, 27, 28, 29, 30, 31, 32, 33, 34, 35, 36, 37, 38, 39, and 40ºC; and in presence of a solvent selected from the list consisting of acetonitrile, acetone, dimethyl sulfoxide (DMSO), acetone, dimethylformamide (DMF), ethanol and methanol, more preferably wherein the solvent is an acetonitrile solvent and wherein the acetonitrile concentration is below 15%.

In another preferred embodiment, alone or in combination with the rest of preferred embodiments, CYP2B10 enzyme is at a concentration between 2 pmol/mL to 50 pmol/mL, more preferably 2 pmol/mL to 15 pmol/mL (including the end values of the range).

In another preferred embodiment, alone or in combination with the rest of preferred embodiments, CYP2B11 enzyme is at a concentration between 1 pmol/mL to 20 pmol/mL, more preferably at a concentration between 1 pmol/mL to 10 pmol/mL (including the end values of the range).

The term "sample" can be defined as part or small quantity of a thing which is considered to be representative of the whole and which is taken or separated for the thing which is considered representative of the whole and which is taken or separated from it for study, analysis or experimentation. Examples of samples include, without limitation to, water, an isolated biological sample (such as sputum, saliva, whole blood, serum, plasma, urine, feces, ejaculate, a buccal or buccal-pharyngeal swab, pleural fluid, peritoneal fluid, pericardial fluid, cerebrospinal fluid, intra-articular fluid, bronchial aspirates, or bronchioalveolar lavages, preferably saliva, whole blood or urine), a food sample, a feed sample, and an environmental sample. Thus, in a preferred embodiment, alone or in combination with the rest of preferred embodiments, the sample is selected from the group consisting of water, an isolated biological sample (such as sputum, saliva, whole blood, serum, plasma, urine, feces, ejaculate, a buccal or buccal-pharyngeal swab, pleural fluid, peritoneal fluid, pericardial fluid, cerebrospinal fluid, intra-articular fluid, bronchial aspirates, or bronchioalveolar lavages, preferably saliva, whole blood or urine), a food sample, a feed sample, and an environmental sample.

The term "isolated biological sample", refers to any sample isolated from a subject and includes, but is not limited to, biological fluids from said subject, obtained by any method known to a person skilled in the art for that purpose. The term "subject" refers to any animal, preferably a mammal, and includes, but is not limited to, domestic and farm animals, primates, and humans.

The term "environmental sample" refers to a sample which comes, or which is isolated from the environment. Examples of environmental samples include, without limitation to, vegetable raw material sample, a fungi raw material sample, as a mushroom sample, a soil or a sediment sample, or a water sample

In a more preferred embodiment, alone or in combination with the rest of preferred embodiments, the sample is selected from the list consisting of, food sample, feed sample, environmental sample, and an isolated biological sample.

The terms "vegetable raw material sample" and "fungi raw material sample", refers to a sample isolated from vegetable/fungi organism and/or from isolated from vegetable/fungi material which can be found in nature, or which is unprocessed/minimally vegetable/fungi processed material.

In a still more preferred embodiment, the food sample comprises mushroom or mushroom fractions.

In another still more preferred embodiment, the environmental sample is selected from the list consisting of a vegetable raw material sample, a fungi raw material sample, as a mushroom sample, a soil or a sediment sample, and a water sample. Even more preferably, the environmental sample is a mushroom sample. The term "mushroom samples" refers to any cultivated or wild fungi. Examples of cultivated fungi includes, without limitation to, common mushrooms/button mushrooms/champignons mushrooms, corn smuts/mexican truffles, enokitake/winter mushrooms, *Fusarium venenatum,* Jew's ears (common name for *Auricularia auricula-judae)*/*hirneola,* Nameko, species belonging to genus *Pleurotus,* Oyester mushrooms, Paddy straw mushroom, Pom-pom blancs/lion's mane, Mushrooms/monkeyhead mushrooms, Shiitake, Shimeji/bunashimeji/beach mushrooms, Snow mushrooms/white jelly mushrooms, Wood blewits/pied bleus, as well as other cultivated fungi. Examples of wild fungi includes, without limitation to, Ceps or Porcini mushrooms, Chanterelles, Hedgehog mushrooms, Horns of plenty/black trumpets, Morels, species belonging to *Tuber* genus, Piemont white truffles, Périgord black truffes, Saint George's mushrooms, Summer truffels (common name for *Tuber Aestivum*)*,* as well as other wild fungi.

The term "an extract thereof" means that an extract of the sample may be also used in the step (a) of the method of the invention. Techniques and methodologies for obtaining extract from a sample are known in the state of the art. However, in a preferred embodiment of the method of the invention, in step (a), an extract of a mushroom is used, more preferably said extract is obtained by an extraction method comprising the following steps:
- an acid treatment of the mushroom sample, preferably comprising the addition of acetic acid, more preferably the addition of acetic acid at a concentration of 2% in a water:acetonitrile solution;
- addition of sodium acetate;
- centrifugation, preferably, wherein the centrifugation comprises centrifuging during 2 to 10 min (including the end values of the range) at a speed range from 1500 to 14000 rpm (including the end values of the range), obtaining a supernatant (organic layer);
- dilution of the supernatant to 40-65% in water, preferably 50% to 60% in water, and
- a solid phase extraction step, preferably wherein the solid phase extraction is performed using C18 cartridge, obtaining at least one mushroom extract.

In a more preferred embodiment, the solid phase extraction, preferably the solid phase extraction using C18 cartridge, comprises:
- passing 1 to 10 mL of the diluted supernatant through the C18 cartridge, obtaining a first elute;
- submitting the first elute to a freeze-out step, preferably at -30 to -10 ºC, more preferably at -20ºC during 20 min;
- evaporation step which comprises the incubation of the extract at 30 to 50ºC under vacuum for 5 to 10 minutes reconstituting with 2 to 10 mL of 5% acetonitrile in water;
- passing the reconstituting volume through a solid phase extraction cartridge, preferably wherein the solid phase extraction cartridge is a hyper crosslinked hydroxylated polystyrene-divinylbenzene copolymer; and
- eluting with 100% acetonitrile, preferably with a volume of acetonitrile of 1 to 3 mL, and passing the resulting volume through a liquid filter cartridge and a PSA cartridge, obtaining a first eluted extract.

Even more preferably, the first eluted extract is submitted to an evaporation step and resuspended in a buffer containing acetonitrile, preferably from 0 to 15% acetonitrile (including the end values of the range).

Even more preferably, the first eluted extract is processed by the following steps:
- acidification of the first eluted extract;
- cleaning step with a cartridge containing NH₂ resin and neutral alumina; and
- filtration and evaporation step, preferably wherein the evaporation step is carried out at 30 to 50ºC under nitrogen flow for 5 to 10 minutes.

In another more preferred embodiment, alone or in combination with the rest of preferred embodiments, the solid phase extraction, preferably the solid phase extraction using C18 cartridge, comprises:
- passing through the cartridge 1 to 5 mL, preferably 2 mL of acetonitrile:water solution (75:25, v/v) with 0.1% acetic acid, obtaining an eluted extract;
- submitting the eluted extract to a freeze-out step preferably at -30 to -10 ºC (including the end values of the range), more preferably at -20ºC during 20 min,
- obtaining a supernatant; and
- eluting the supernatant through a cartridge containing NH₂ resin and neutral alumina; obtaining a second eluted extract.

Even more preferably, the second eluted extract is submitted to an evaporation step and resuspended in a buffer containing 10% acetonitrile, preferably wherein the evaporation step is carried out at 30 to 50ºC under nitrogen flow for 5 to 10 minutes.

In another more preferred embodiment, alone or in combination with the rest of preferred embodiments, the solid phase extraction, preferably the solid phase extraction using C18 cartridge, comprises:
- passing through the cartridge 1 to 5 mL, preferably 2 mL, of 100% acetonitrile with 0.1 % of acetic acid; and
- cleaning with a PSA and neutral alumina cartridge, obtaining a third eluted extract.

Even more preferably, the third eluted extract is submitted to an evaporation and reconstitution in a working buffer. As used herein, "working buffer" refers to a buffer which allows to reach a pH between 6 to 9. Preferably, the working buffer is phosphate buffer at a concentration between 10 to 200 mM (including the end values of the range).

In the present invention, the CYP2B11 and/or CYP2B10 enzymes, can be used in combination with other enzymes, which are also inhibited by pesticides. Examples of enzymes that may be inhibited by pesticides include, without limitation to acid phosphatase enzyme, acetylcholinesterase enzyme, aldehyde dehydrogenase enzyme, alkaline phosphatase enzyme, glutathione S-transferase enzyme, laccase enzyme, other enzymes belonging to CYP450 family (CYP450 enzyme/s other/s than CYP2B10 and CYP2B11, already included), methyl parathion hydrolase enzyme, organophosphorus hydrolase enzyme, peroxidase enzymes, tyrosinase enzyme and urease enzyme. Thus, in a preferred embodiment of the method of the invention, alone or in combination with the rest of preferred embodiments, the step (a) further comprises contacting the sample or an extract thereof with, at least, one enzyme selected from the list consisting of acid phosphatase enzyme, acetylcholinesterase enzyme, aldehyde dehydrogenase enzyme, alkaline phosphatase enzyme, glutathione S-transferase enzyme, laccase enzyme, CYP450 enzyme (CYP450 enzyme/s other/s than CYP2B10 and CYP2B11, already included), methyl parathion hydrolase enzyme, organophosphorus hydrolase enzyme, peroxidase enzymes, tyrosinase enzyme, urease enzyme, and any combination thereof.

### Step (b) of the method of the invention

A second step of the method of the invention [step (b)], comprises the addition of, at least, one enzyme reporter substrate.

The term "enzyme reporter substrate", as used herein, refers to any molecule, compound or reagent able to interact with the enzyme/s used in the step (a) of the method, generating or producing a product by the catalytic action of the enzyme, which is detectable.

The phrase "at least one enzyme reporter substrate", as used herein, refers to one or more enzyme reporter substrate, used in the step (b) of the method of the invention.

In a preferred embodiment of the method of the invention, alone or in combination with the rest of preferred embodiments, the at least one enzyme reporter substrate is a fluorogenic substrate, preferably a fluorescein derivative, a coumarin derivative and/or a resorufin derivative.

Examples of fluorescein derivatives are dibenzylfluorescein (DBF), di(benzyloxymethyl)fluorescein (DBOMF), benzyloxy-methyl- fluorescein (BOMF), -3-O-methylfluorescein (OMF), monobenzylfluorescein (MBF) and diethoxyfluorescein (DEF). Examples of coumarin derivatives are 7-Ethoxymethoxy-3- cyanocoumarin (EOMCC), 7-benzyloxymethyloxy-3-cyanocoumarin (BOMCC), 7-p- methoxy-benzyloxy-4-trifluoro-coumarin (MOBFC), 3-[2-(N,N-diethyl-N-methylamino)ethyl]-7-methoxy-4-methylcoumarin (AMMC), 3-[2-(N,N-diethyl-N-methylamino)ethyl]-7-methoxy-4-trifluoromethylcoumarin (MeAMFC), 7-methoxy-3- cyanocoumarin (CMC), 7-ethoxy-3-cyanocoumarin (CEC), 7-methoxy-4-trifluoromethylcoumarin (MOFC), 7-benzyloxy-4-trifluoromethylcoumarin (BFC), 7-Benzyloxy-3-cyanocoumarin (BCC), 7-ethoxy-4-trifluoromethylcoumarin (EFC) and 7-hydroxy-4-trifluoromethylcoumarin (HFC). Examples of resorufin derivatives are benzyloxyresorufin (BR), benzyloxy-methyl-resorufin (BOMR), n-octyloxy-methyl-resorufin (OOMR) and ethoxyresorufin (ER).

Other examples of enzyme reporter substrates that may be used in the present invention, without being limiting, are diazepam, bupropion, ketamine, midazolam, propofol, tramadol, nicotine, N'-hydroxymethyl-norcotinine or cotinine.

Thus, in a preferred embodiment of the method of the invention, the step (b) comprises the addition of, at least, one enzyme reporter substrate is selected from the list consisting of DBF, DBOMF, BOMF, OMF, MBF, DEF, EOMCC, BOMCC, MOBFC, AMMC, MeAMFC, CMC, CEC, MOFC, BFC, BCC, EFC, HFC, BR, BOMR, OOMR, ER, diazepam, bupropion, ketamine, midazolam, propofol, tramadol, nicotine, N'-hydroxymethyl-norcotinine, cotinine, and any combination thereof.

In a more preferred embodiment of the method of the invention, when the step (a) comprises contacting the sample or an extract thereof, with a CYP2B11 enzyme, the enzyme reporter substrate is selected from the list consisting of BOMCC, EOMCC, MOBFC, MOFC, diazepam and bupropion, ketamine, midazolam, propofol, tramadol, and any combination thereof.

In another more preferred embodiment of the method of the invention, when the step (a) comprises contacting the sample or an extract thereof, with a CYP2B10 enzyme, the enzyme reporter substrate is selected from the list consisting of MOBFC, EOMCC, BOMCC, MOFC, bupropion, (S)-nicotine, N'-hydroxymethyl-norcotinine, cotinine and any combination thereof.

In another more preferred embodiment of the method of the invention when the step (a) comprises contacting the sample or an extract thereof, with a CYP2B11 enzyme and a CYP2B10 enzyme, the enzyme reporter substrates are BOMCC and MOBFC.

Moreover, as it has been previously mentioned, the CYP2B11 and/or CYP2B10 enzymes, can be used in combination with other enzymes in the step (a) of the method of the invention.

In this step (b), the addition of, at least, one enzyme reporter substrate includes the incubation at suitable conditions to allow a catalytic reaction, between the substrate and the enzyme with which the sample is contacted in the step (a), generating a detectable signal. This detectable signal is detected and measured in the step (c) of the method of the invention.

In a preferred embodiment of the method of the invention, alone or in combination with the rest of preferred embodiments, the step (b) comprises the addition of, at least, one enzyme reporter substrate and the incubation at suitable conditions, wherein the suitable conditions comprise the incubation during 5 to 60 min (including the end values of the range).

In another preferred embodiment of the method of the invention, alone or in combination with the rest of preferred embodiments, the step (b) comprises the addition of, at least, one enzyme reporter substrate and the incubation at suitable conditions, wherein the suitable conditions comprise the incubation at a temperature of 20 ºC to 40 ºC (including the end values of the range), preferably at a temperature selected from the list consisting of 20, 21, 22, 23, 24, 25, 26, 27, 28, 29, 30, 31, 32, 33, 34, 35, 36, 37, 38, 39, and 40ºC.

In a more preferred embodiment, alone or in combination with the rest of preferred embodiments, step (b) comprises the addition of, at least, one enzyme reporter substrate and the incubation at suitable conditions, wherein the suitable conditions comprise the incubation during 5 to 60 min (including the end values of the range), and at a temperature of 20 ºC to 40 ºC (including the end values of the range).even more preferably at a temperature selected from the list consisting of 20, 21, 22, 23, 24, 25, 26, 27, 28, 29, 30, 31, 32, 33, 34, 35, 36, 37, 38, 39, and 40ºC.

### Step (c) of the method of the invention

A third step, the step (c) of the method of the invention, comprises the detection and measurement of a detectable signal.

As it has been previously mentioned, in the step (b) of the method of the invention, the enzyme/s reporter substrate/s addition allow a catalytic reaction between the substrate and the enzyme, generating a detectable signal.

The detection and measurement of the detectable signal can be carried out by techniques known in the state of the art. In a preferred embodiment of the method of the invention, the detectable signal is detected and measured by optical techniques (fluorometric technique, such as fluorometry; or other optical techniques such as colorimetry, spectroscopy and surface plasmon resonance), electrochemical techniques (such as impedance, potentiometry, amperometry, coulometry and fieldeffect transistors), magnetic techniques (such as paramagnetic techniques, magnetooptical properties based techniques), mass-based techniques (such as quartz crystal microbalance, surface acoustic wave sensors), thermistor-based techniques (such as calorimetry) or chromatographic techniques (such as liquid chromatography-mass spectrometry (LC-MS/MS)). Preferably, the detectable signal is detected and measured by fluorimetry or by an electrochemical technique.

In a more preferred embodiment of the method of the invention, alone or in combination with the rest of preferred embodiments, when the detectable signal is detected and measured by fluorimetry, the enzyme reporter substrate is a fluorogenic substrate. In this preferred embodiment, the fluorogenic substrate is converted to a fluorescent product detectable by fluorometric techniques, and which is generated by the catalytic reaction with the enzyme used in the step (a).

In another more preferred embodiment of the method of the invention, alone or in combination with the rest of preferred embodiments, when the detectable signal is detected and measured by an electrochemical technique, the enzyme reporter substrate is electroactive. In this preferred embodiment, the electroactive substrate is converted to a product detectable by electrochemical techniques, and which is generated by the catalytic reaction with the enzyme used in the step (a).

In another more preferred embodiment of the method of the invention, alone or in combination with the rest of preferred embodiments, when the detectable signal is detected and measured by an electrochemical technique, the enzyme reporter substrate is non-electroactive but the product resultant from the catalytic reaction between the substrate and the enzyme is electroactive. In this preferred embodiment, the enzyme reporter substrate is converted to an electroactive product detectable by electrochemical techniques, and which is generated by the catalytic reaction with the enzyme used in the step (a).

In another more preferred embodiment of the method of the invention, alone or in combination with the rest of preferred embodiments, when the detectable signal is detected and measured by an electrochemical technique, the enzyme reporter substrate and product are non-electroactive, but the resultant catalytic reaction can be monitored by the direct redox reaction within the active centre of the enzyme and the electrode surface. In this preferred embodiment, the enzyme non-electroactive substrate is converted to a non-electroactive product detectable by electrochemical techniques, and which is generated by the direct redox reaction between the catalytic centre of the enzyme and the electrode with the enzyme used in the step (a).

As used herein, the singular forms "a," "an" and "the", along with the term "detectable signal", "enzyme reporter substrate" can include also their corresponding plural forms. For instance, when 2, 3, 4, 5 or more different enzyme reporter substrates are added in the step (b), 2, 3, 4, 5 or more different detectable signals can be detected and measured in the step (c) of the method of the invention.

### Step (d) of the method of the invention

A fourth step, the step (d) of the method of the invention, comprises the determination of pesticide presence in the sample by comparing the measured detectable signal with a reference value, preferably, wherein the reference value is a signal from a reference sample.

The reference sample can be a sample without the pesticide/s to be detected or with a known concentration of said pesticide/s, preferably wherein the known concentration of said pesticide/s is below the regulatory levels or present at trace concentrations.

Since the invention is based on specific enzymes inhibition by pesticides, a measured detectable signal significantly reduced or lower than the signal from a reference sample indicates pesticide presence in the sample.

In the present invention, by comparing the measured detectable signal with a reference value, an enzyme inhibition rate can be calculated as follows: $Enzyme inhibition rate \left(%\right) = 100 - \left(\frac{Measured detectable signal}{Reference value}\times 100\right)$

Thus, in a preferred embodiment, alone or in combination with the rest of preferred embodiments, an enzyme inhibition rate higher than 10%, more preferably, higher than 20%, indicates pesticide presence in the sample.

As used herein, the singular forms "a," "an" and "the", along with the term "detectable signal" and "pesticide" can include also their corresponding plural forms. For instance, when 2, 3, 4, 5 or more different detectable signals are measured, 2, 3, 4, 5 or more different pesticides can be present in the sample.

In a preferred embodiment of the method of the invention, alone or in combination with the rest of preferred embodiments, the pesticide is one or more pesticides selected from the list consisting of, benalaxyl, captan, chlorpyrifos, chlorpyrifos methyl, chlorothalonil, cyazofamid, cypermethrin, DDT, deltamethrin, diazinon, dicofol, dimethoate, epoxiconazole, fenbuconazole, fenitrothion, folpet, mancozeb, meptyldinocap, metrafenone, myclobutanil, penconazole, phosmet, piperonyl butoxide, pirimiphos methyl, prochloraz, procymidone, propiconazole, simazine and any combination thereof.

In addition, when the method of the invention comprises the detection of one pesticide, the method of the invention may include an additional step, the step (e), which comprises determining the pesticide concentration. In a preferred embodiment, the determination of the pesticide concentration comprises comparing the measured detectable signal with a calibration curve. In this embodiment, in order to determine the pesticide concentration, a calibration is carried out and the enzyme inhibition rate is calculated versus a reference value. Representing the enzyme inhibition rate versus the concentration, provides a calibration that allows to infer the concentration of the compound in the sample.

The definitions and preferred embodiments described for the first aspect of the invention are applicable to the rest of the aspects of the invention.

### Kit of the invention and uses thereof

The implementation of the uses and method of the invention are based on inhibition of specific enzymes by pesticide/s and the generation of a detectable signal produced by the catalytic action of the enzyme/s to enzyme reporter substrate/s. Thus, other aspect of the present invention relates to a kit, hereinafter the "kit of the invention", comprising a CYP2B11 enzyme, a CYP2B10 enzyme, and at least one enzyme reporter substrate.

The kit of the invention is suitable for pesticide detection. Thus, a preferred embodiment provides the kit of the invention, wherein the kit of the invention is for pesticide detection.

Furthermore, the kit of the invention is suitable for carrying out the method according to the first aspect of the invention, including any one of its preferred embodiments. Thus, another preferred embodiment provides the kit of the invention for carrying out the method of the invention.

Thus, the kit of the invention is suitable for detecting pesticides, based on the inhibition of specific enzymes by pesticide/s, particularly the inhibition of CYP2B11 enzyme and/or CYP2B10 enzyme. Additionally, the kit of the invention may further comprise other enzymes which are also inhibited by pesticides. A preferred embodiment, alone or in combination with the rest of preferred embodiments, provides the kit of the invention which further comprises, at least, one enzyme selected from the list consisting of acid phosphatase enzyme, acetylcholinesterase enzyme, aldehyde dehydrogenase enzyme, alkaline phosphatase enzyme, glutathione S-transferase enzyme, laccase enzyme, other enzymes belonging to CYP450 family (CYP450 enzyme/s other/s than CYP2B10 and CYP2B11, already included), methyl parathion hydrolase enzyme, organophosphorus hydrolase enzyme, peroxidase enzymes, tyrosinase enzyme, urease enzyme, and any combination thereof.

A third element in the kit of the invention is "at least one enzyme reporter substrate", which interacts with the enzyme/s of the kit of the invention, and which allows the generation or production of a detectable signal mediated by the catalytic action of the enzyme.

The phrase "at least one enzyme reporter substrate", as used herein, refers to one or more enzyme reporter substrate.

In a preferred embodiment of the kit of the invention, alone or in combination with the rest of preferred embodiments, the enzyme reporter substrate is a fluorogenic substrate, preferably a fluorescein derivative, a coumarin derivative and/or a resorufin derivative.

Examples of fluorescein derivatives are dibenzylfluorescein (DBF), di(benzyloxymethyl)fluorescein (DBOMF), benzyloxy-methyl- fluorescein (BOMF), -3-O-methylfluorescein (OMF), monobenzylfluorescein (MBF) and diethoxyfluorescein (DEF). Examples of coumarin derivatives are 7-Ethoxymethoxy-3- cyanocoumarin (EOMCC), 7-benzyloxymethyloxy-3-cyanocoumarin (BOMCC), 7-p- methoxy-benzyloxy-4-trifluoro-coumarin (MOBFC), 3-[2-(N,N-diethyl-N-methylamino)ethyl]-7-methoxy-4-methylcoumarin (AMMC), 3-[2-(N,N-diethyl-N-methylamino)ethyl]-7-methoxy-4-trifluoromethylcoumarin (MeAMFC), 7-methoxy-3- cyanocoumarin (CMC), 7-ethoxy-3-cyano-coumarin (CEC), 7-methoxy-4-trifluoromethylcoumarin (MOFC), 7-benzyloxy-4-trifluoromethylcoumarin (BFC), 7-Benzyloxy-3-cyanocoumarin (BCC), 7-ethoxy-4-trifluoromethylcoumarin (EFC) and 7-hydroxy-4-trifluoromethylcoumarin (HFC). Examples of resorufin derivatives are benzyloxyresorufin (BR), benzyloxy-methyl-resorufin (BOMR), n-octyloxy-methyl-resorufin (OOMR) and ethoxyresorufin (ER).

Other examples of enzyme reporter substrates that may be used in the present invention, without being limiting, are diazepam, bupropion, ketamine, midazolam, propofol, tramadol, nicotine, N'-hydroxymethyl-norcotinine or cotinine.

In a more preferred embodiment of the kit of the invention, the enzyme reporter substrate is selected from the list consisting of DBF, DBOMF, BOMF, OMF, MBF, DEF, EOMCC, BOMCC, MOBFC, AMMC, MeAMFC, CMC, CEC, MFC, BFC, BCC, EFC, HFC, BR, BOMR, OOMR, ER, diazepam, bupropion, ketamine, midazolam, propofol, tramadol, nicotine, N'-hydroxymethyl-norcotinine, cotinine, and any combination thereof.

In another still more preferred embodiment, the enzyme reporter substrates are BOMCC and MOBFC.

The terms "CYB2B11 enzyme", "CYP2B10 enzyme", and "enzyme reporter substrate", have been described in the previous aspect of the invention, and its definitions, as well as its preferred embodiments, apply equally to kit of the invention and uses thereof.

In addition, as a person skilled in the art knows, the kit may comprise other components useful in the implementation of the present invention, such as, buffer solutions, delivery vehicles, material carriers, positive and/or negative control components, etc. In addition to the aforementioned components, the kits may also include instructions for practicing the subject matter of the invention. These instructions may be present in the aforementioned kits in a variety of forms, one or more of which may be present in the kit. One form in which these instructions may be present is as printed information on a suitable medium or substrate, for example a sheet or sheets of paper on which the information is printed, on the kit packaging, on a package insert, etc. Another medium would be a computer readable medium, e.g. CD, USB, etc., on which the information has been recorded. Another medium that may be present is a website address that can be used via the Internet to access the information at a remote site. Any convenient medium may be present in the kits.

The kit of the invention can be used for pesticide detection in a sample, and in the method of the invention.

Thus, other aspect of the invention relates to the use of the kit of the invention in the method of the invention. That is to say, the use of the kit of the invention in the method according to any one of the embodiments of the first aspect of the invention.

In other aspect, the invention relates to the use of the kit of the invention for pesticide detection in a sample or an extract thereof, hereinafter the "use of the kit of the invention".

The terms "pesticide" and "detection" have been already described in the previous aspect of the present invention and apply equally, including the pesticide detection preferred embodiments, to the kit of the invention and uses thereof. As mentioned in the previous aspect of the invention, multianalyte detection, in the present invention the simultaneous detection of multiple pesticides, is also considered.

In a preferred embodiment, alone or in combination with the rest of preferred embodiments, pesticide detection comprises the detection of 2, 3, 4, 5, 6, 7, 8, 9, 10, 11, 12, 13, 14, 15 or more pesticides.

Another preferred embodiment, alone or in combination with the rest of preferred embodiments, provides the use of the kit of the invention wherein the pesticide detection comprises the detection of at least one pesticide, wherein the pesticide belongs to a family of pesticides selected from the list consisting of amide, aryl phenyl ketone, azole, benzophenone, dicarboximide, dinitrophenol, dithiocarbamate, organobromide, organochlorine, organophosphate, phthalimide, pyrethroid, triazine, synergists molecules and any combination thereof. In the use of the kit of the invention, when the pesticide detection comprises the detection of 2, 3, 4, 5, 6, 7, 8, 9, 10, 11, 12, 13, 14, 15 or more pesticides, the pesticides can be from any of the families above mentioned, and any combination thereof.

Another preferred embodiment, alone or in combination with the rest of preferred embodiments, provides the use of the kit of the invention wherein the pesticide detection comprises the detection of at least one pesticide selected from the list consisting of benalaxyl, captan, chlorpyrifos, chlorpyrifos methyl, chlorothalonil, cyazofamid, cypermethrin, DDT, deltamethrin, diazinon, dicofol, dimethoate, epoxiconazole, fenbuconazole, fenitrothion, folpet, mancozeb, meptyldinocap, metrafenone, myclobutanil, penconazole, phosmet, piperonyl butoxide, pirimiphos methyl, prochloraz, procymidone, propiconazole, simazine and any combination thereof.

In another more preferred embodiment, when the kit of the invention comprises a CYP2B11 enzyme, the pesticide detection comprises the detection of at least one pesticide belonging to the pesticide family selected from the list consisting of amide, aryl phenyl ketone, azole, benzophenone, dicarboximide, dinitrophenol, dithiocarbamate, organobromide, organochlorine, organophosphate, phthalimide, phthalimide/organophosphate, pyrethroid, triazine, synergists molecules and any combination thereof. More preferably, when the kit of the invention comprises a CYP2B11 enzyme, the pesticide detection comprises the detection of at least one pesticide selected from the list consisting of benalaxyl, captan, chlorpyrifos, chlorpyrifos methyl, chlorothalonil, cyazofamid, cypermethrin, DDT, deltamethrin, diazinon, dicofol, dimethoate, epoxiconazole, fenbuconazole, fenitrothion, folpet, mancozeb, meptyldinocap, metrafenone, myclobutanil, penconazole, phosmet, piperonyl butoxide, pirimiphos methyl, prochloraz, procymidone, propiconazole, simazine and any combination thereof.

In another more preferred embodiment, when the kit of the invention comprises a CYP2B10 enzyme, the pesticide detection comprises the detection of at least one pesticide belonging to the pesticide family selected from the list consisting of azole, organochlorine, organophosphate, phthalimide, phthalimide/organophosphate, triazine, and any combination thereof. More preferably, when the kit of the invention comprises a CYP2B10 enzyme, the pesticide detection comprises the detection of at least one pesticide selected from the list consisting of captan, chlorpyrifos, chlorpyrifos methyl, diazinon, dicofol, fenbuconazole, fenitrothion, folpet, myclobutanil, penconazole, phosmet, pirimiphos methyl, prochloraz, propiconazole, simazine and any combination thereof.

In a preferred embodiment, alone or in combination with the rest of preferred embodiments, when the kit of the invention comprises a CYP2B11 enzyme and a CYP2B10 enzyme, the pesticide detection comprises the detection of at least one pesticide belonging to the pesticide family selected from the list consisting of amide, aryl phenyl ketone, azole, benzophenone, dicarboximide, dinitrophenol, dithiocarbamate, organobromide, organochlorine, organophosphate, phthalimide, pyrethroid, triazine, synergists molecules and any combination thereof.

More preferably, when the kit of the invention comprises a CYP2B11 enzyme and a CYP2B10 enzyme the pesticide detection comprises the detection of at least one pesticide selected from the list consisting of benalaxyl, captan, chlorpyrifos, chlorpyrifos methyl, chlorothalonil, cyazofamid, cypermethrin, DDT, deltamethrin, diazinon, dicofol, dimethoate, epoxiconazole, fenbuconazole, fenitrothion, folpet, mancozeb, meptyldinocap, metrafenone, myclobutanil, penconazole, phosmet, piperonyl butoxide, pirimiphos methyl, prochloraz, procymidone, propiconazole, simazine and any combination thereof.

In another preferred embodiment of the use of the kit of the invention, alone or in combination with the rest of preferred embodiments, when the sample is a mushroom sample or a mushroom extract, the pesticide detection comprises the detection of, at least, one pesticide selected from the list consisting of prochloraz, procymidone, chlorothalonil, dicofol, chlorpyrifos, chlorpyrifos-methyl, diazinon, piperonyl butoxide, cypermethrin, metrafenone and/or deltamethrin.

As mentioned in the previous aspect of the invention, examples of samples include, without limitation to, water, an isolated biological sample (such as sputum, saliva, whole blood, serum, plasma, urine, feces, ejaculate, a buccal or buccal-pharyngeal swab, pleural fluid, peritoneal fluid, pericardial fluid, cerebrospinal fluid, intra-articular fluid, bronchial aspirates, or bronchioalveolar lavages, preferably saliva, whole blood or urine), a food sample, a feed sample, or an environmental sample.

In a preferred embodiment, alone or in combination with the rest of preferred embodiments, the sample is selected from the group consisting of water, an isolated biological sample (such as sputum, saliva, whole blood, serum, plasma, urine, feces, ejaculate, a buccal or buccal-pharyngeal swab, pleural fluid, peritoneal fluid, pericardial fluid, cerebrospinal fluid, intra-articular fluid, bronchial aspirates, or bronchioalveolar lavages, preferably saliva, whole blood or urine), a food sample, a feed sample, and an environmental sample. Furthermore, in the present invention, the sample may also be an extract of the sample, including an extract of any of the above-mentioned samples. The term "an extract thereof", have been described in the previous aspect of the invention, and its definition and preferred embodiments apply equally herein. Preferably the extract is a mushroom extract.

In a more preferred embodiment, alone or in combination with the rest of preferred embodiments, the sample is selected from the list consisting of, food sample, feed sample, environmental sample, and an isolated biological sample.

In a still more preferred embodiment, the food sample comprises mushroom or mushroom fractions.

In another still more preferred embodiment, the environmental sample is selected from the list consisting of a vegetable raw material sample, a fungi raw material sample, as a mushroom sample, a soil or a sediment sample, and a water sample. Even more preferably, the environmental sample is a mushroom sample.

The terms used to define the kit of the invention and uses thereof, have already been described in the previous aspect of the present invention and apply equally, as well as its preferred embodiments, to the kit of the invention and uses thereof.

### Use of the invention

As explained before, CYP2B11 enzyme and/or CYP2B10 enzyme, alone or in combination with other enzymes, have application in pesticide detection.

Thus, other aspect of the present invention relates to the use of, at least, one CYP450 enzyme selected from the list consisting of CYP2B11 enzyme and CYP2B10 enzyme for pesticide detection in a sample, hereinafter the "use of the invention".

In the present invention, the CYP2B11 and/or CYP2B10 enzymes, can be used in combination with other enzymes, which are also inhibited by pesticides. Thus, a preferred embodiment, alone or in combination with the rest of preferred embodiments, provides the use of the invention, which further comprises the use of, at least, one enzyme selected from the list consisting of acid phosphatase enzyme, acetylcholinesterase enzyme, aldehyde dehydrogenase enzyme, alkaline phosphatase enzyme, glutathione S-transferase enzyme, laccase enzyme, other enzymes belonging to CYP450 family (CYP450 enzyme/s other/s than CYP2B10 and CYP2B11, already included), methyl parathion hydrolase enzyme, organophosphorus hydrolase enzyme, peroxidase enzymes, tyrosinase enzyme, urease enzyme, and any combination thereof.

As specifically included for other aspects of the present invention, all their combinations are applicable to the use of the invention. Moreover, the terms "CYB2B11 enzyme" and "CYP2B10 enzyme", have been described in the first aspect of the invention, and its definitions, as well as its preferred embodiments, apply equally to the use of the invention

Furthermore, for pesticide detection can be also used at least one enzyme reporter substrate, which interacts with the previous mentioned enzyme/s, and which allows the generation or production of a detectable signal mediated by the catalytic action of the enzyme. The phrase "at least one enzyme reporter substrate", as used herein, refers to one or more enzyme reporter substrate. The term "enzyme reporter substrate" have been previously described, and its definition, as well as its preferred embodiments (including its particular combinations with the enzymes) are applicable to the use of the invention.

Thus, in a preferred embodiment of the use of the invention, alone or in combination with the rest of preferred embodiments, the enzyme reporter substrate is a fluorogenic substrate, preferably as a fluorescein derivative, a coumarin derivative and/or a resorufin derivative.

In a more preferred embodiment of the use of the invention, the enzyme reporter substrate is selected from the list consisting of DBF, DBOMF, BOMF, OMF, MBF, DEF, EOMCC, BOMCC, MOBFC, AMMC, MeAMFC, CMC, CEC, MFC, BFC, BCC, EFC, HFC, BR, BOMR, OOMR, ER, diazepam, bupropion, ketamine, midazolam, propofol, tramadol, nicotine, N'-hydroxymethyl-norcotinine, cotinine, and any combination thereof.

In a still more preferred embodiment, when the use of the invention comprises a CYP2B11 enzyme, the enzyme reporter substrate is selected from the list consisting of BOMCC, EOMCC, MOBFC, MOFC, diazepam and bupropion, ketamine, midazolam, propofol, tramadol, and any combination thereof.

In another still more preferred embodiment, when the use of the invention comprises a CYP2B10 enzyme, the enzyme reporter substrate is selected from the list consisting of MOBFC, EOMCC, BOMCC, MOFC, bupropion, (S)-nicotine, N'-hydroxymethylnorcotinine, cotinine, and any combination thereof.

In another still more preferred embodiment, when the use of the invention comprises a CYP2B11 enzyme and a CYP2B10 enzyme, the enzyme reporter substrates are BOMCC and MOBFC.

A higher enzyme inhibition in the sample than in a reference sample, indicates pesticide presence in the sample. As it has been explained in the method of the invention, by comparing a measured detectable signal in a sample when the enzymes of the invention are used, with a reference value, an enzyme inhibition rate can be calculated. Thus, in a preferred embodiment of the use of the invention, alone or in combination with the rest of preferred embodiments, a higher enzyme inhibition rate in the sample than the enzyme inhibition rate in the reference sample, indicates pesticide presence in the sample.

The terms "reference value" or "enzyme inhibition rate" have been previously defined and/or explained in the method of the invention, applying equally to the use of the invention.

As used herein, the singular forms "a," "an" and "the", along with the term "detectable signal" and "pesticide" can include also their corresponding plural forms. For instance, when 2, 3, 4, 5 or more different detectable signals are measured, 2, 3, 4, 5 or more different pesticides may be present in the sample.

The term "sample" have been already described in the previous aspect of the present invention and its definition as well as its preferred embodiments, apply equally to the use of the invention. In a preferred embodiment of the use of the invention, the sample is selected from the list consisting of, food sample, feed sample, environmental sample, and an isolated biological sample. Furthermore, in the present invention, the sample may also be an extract of the sample, including an extract of any of the above-mentioned samples. The term "an extract thereof", have been described in the previous aspects of the invention, and its definition and preferred embodiments apply equally herein. Preferably the extract is a mushroom extract.

The reference sample can be a sample without the pesticide/s to be detected or with a known concentration of said pesticide/s.

The terms "pesticide" and "detection" have been already described in the previous aspects of the present invention, as well the pesticide detection preferred embodiments. The preferred embodiments of pesticide detection apply equally to the use of the invention.

In a preferred embodiment of the use of the invention, alone or in combination with the rest of preferred embodiments, pesticide detection comprises the detection of 2, 3, 4, 5, 6, 7, 8, 9, 10, 11, 12, 13, 14, 15 or more pesticides.

Another preferred embodiment, alone or in combination with the rest of preferred embodiments, provides the use of the invention wherein the pesticide detection comprises the detection of, at least, one pesticide, wherein the pesticide belongs to a family of pesticides selected from the list consisting of amide, aryl phenyl ketone, azole, benzophenone, dicarboximide, dinitrophenol, dithiocarbamate, organobromide, organochlorine, organophosphate, phthalimide, pyrethroid, triazine, synergists molecules and any combination thereof. In the use of the invention, when the pesticide detection comprises the detection of 2, 3, 4, 5, 6, 7, 8, 9, 10, 11, 12, 13, 14, 15 or more pesticides, the pesticides can be from any of the families above mentioned, and any combination thereof.

Another preferred embodiment, alone or in combination with the rest of preferred embodiments, provides the use of the invention wherein the pesticide detection comprises the detection of, at least, one pesticide selected from the list consisting of benalaxyl, captan, chlorpyrifos, chlorpyrifos methyl, chlorothalonil, cyazofamid, cypermethrin, DDT, deltamethrin, diazinon, dicofol, dimethoate, epoxiconazole, fenbuconazole, fenitrothion, folpet, mancozeb, meptyldinocap, metrafenone, myclobutanil, penconazole, phosmet, piperonyl butoxide, pirimiphos methyl, prochloraz, procymidone, propiconazole, simazine, and any combination thereof.

In another more preferred embodiment, when the use of the invention comprises a CYP2B11 enzyme, the pesticide detection comprises the detection of at least one pesticide belonging to the pesticide family selected from the list consisting of amide, aryl phenyl ketone, azole, benzophenone, dicarboximide, dinitrophenol, dithiocarbamate, organobromide, organochlorine, organophosphate, phthalimide, phthalimide/organophosphate, pyrethroid, triazine, synergists molecules and any combination thereof. More preferably, when the kit of the invention comprises a CYP2B11 enzyme, the pesticide detection comprises the detection of at least one pesticide selected from the list consisting of benalaxyl, captan, chlorpyrifos, chlorpyrifos methyl, chlorothalonil, cyazofamid, cypermethrin, DDT, deltamethrin, diazinon, dicofol, dimethoate, epoxiconazole, fenbuconazole, fenitrothion, folpet, mancozeb, meptyldinocap, metrafenone, myclobutanil, penconazole, phosmet, piperonyl butoxide, pirimiphos methyl, prochloraz, procymidone, propiconazole, simazine and any combination thereof.

In another more preferred embodiment, when the use of the invention comprises a CYP2B10 enzyme, the pesticide detection comprises the detection of, at least, one pesticide belonging to the pesticide family selected from the list consisting of azole, organochlorine, organophosphate, phthalimide, phthalimide/organophosphate, triazine, and any combination thereof. More preferably, when the use of the invention comprises a CYP2B10 enzyme, the pesticide detection comprises the detection of, at least, one pesticide selected from the list consisting of captan, chlorpyrifos, chlorpyrifos methyl, diazinon, dicofol, fenbuconazole, fenitrothion, folpet, myclobutanil, penconazole, phosmet, pirimiphos methyl, prochloraz, propiconazole, simazine and any combination thereof.

In a preferred embodiment, alone or in combination with the rest of preferred embodiments, when the use of the invention comprises a CYP2B11 enzyme and a CYP2B10 enzyme, the pesticide detection comprises the detection of at least one pesticide belonging to the pesticide family selected from the list consisting of amide, aryl phenyl ketone, azole, benzophenone, dicarboximide, dinitrophenol, dithiocarbamate, organobromide, organochlorine, organophosphate, phthalimide, pyrethroid, triazine, synergists molecules and any combination thereof.

More preferably, when the use of the invention comprises a CYP2B11 enzyme and a CYP2B10 enzyme the pesticide detection comprises the detection of at least one pesticide selected from the list consisting of benalaxyl, captan, chlorpyrifos, chlorpyrifos methyl, chlorothalonil, cyazofamid, cypermethrin, DDT, deltamethrin, diazinon, dicofol, dimethoate, epoxiconazole, fenbuconazole, fenitrothion, folpet, mancozeb, meptyldinocap, metrafenone, myclobutanil, penconazole, phosmet, piperonyl butoxide, pirimiphos methyl, prochloraz, procymidone, propiconazole, simazine and any combination thereof.

In another preferred embodiment of the use of the of the invention, alone or in combination with the rest of preferred embodiments, when the sample is a mushroom sample or a mushroom extract, the pesticide detection comprises the detection of, at least, one pesticide selected from the list consisting of prochloraz, procymidone, chlorothalonil, dicofol, chlorpyrifos, chlorpyrifos-methyl, diazinon, piperonyl butoxide, cypermethrin, metrafenone and/or deltamethrin.

### DESCRIPTION OF THE DRAWINGS

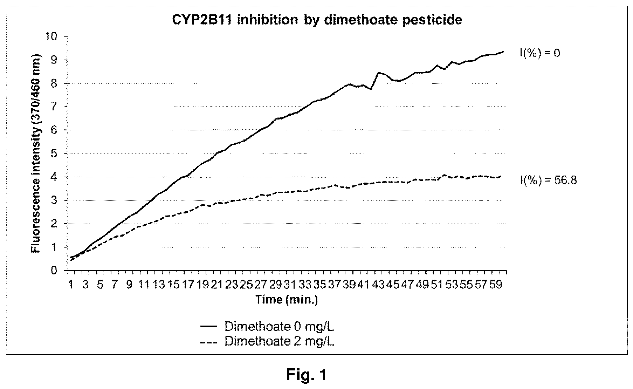

**Fig. 1****.** Fluorescence intensity and inhibition percentages registered for CYP2B11 in presence and in absence of 2 mg/L dimethoate pesticide.

### Examples

### Example 1 - Pesticides screening using CYP2B10 inhibition assay

An inhibition assay was performed using the enzyme CYP2B10 by contacting it to 200 µg/L and 400 µg/L of chlorpyrifos-ethyl, chlorpyrifos-methyl, prochloraz, diazinon and dicofol.

This assay was performed in a 96-well plate by adding 50 µL of chemical standard of pesticides in duplicate, previously prepared in assay buffer with 10% acetonitrile. Enzyme mixture consisted of 15 pmol/mL CYP2B10 (Cypex Limited) in the assay buffer: 75 mM potassium phosphate supplemented with 5 mM MgCl₂, pH=8.0, containing 333 mM Glucose-6-phosphate and 30 U/mL Glucose-6-phosphate dehydrogenase. After preparation, 50 µL of enzyme mixture were added to each plate and incubated for 10 minutes at 37°C. After incubation, 10 µL of a substrate mixture containing 30 µM NADP⁺ and 10 µM MOBFC in the phosphate buffer was added to the wells. The plate was incubated for 60 minutes at 37°C, and the fluorescence was monitored using 370/520 nm as excitation and emission wavelengths, respectively. A blank sample containing buffer solution was measured in same conditions and used to calculate the inhibition of the enzyme activity that was associated with the presence of pesticides concentration.

**Table 1. Inhibition percentage (I (%)) calculated for CYP2B10 reactions in presence of 200 µg/L and 400 µg/L of chlorpyrifos-ethyl, chlorpyrifos-methyl, prochloraz, diazinon and dicofol.**

| **Pesticides** | **I (%) at 200 µg/L** | **I (%) at 400 µg/L** |
|---|---|---|
| Chlorpyrifos-ethyl | 26.8 | 46.8 |
| Chlorpyrifos-methyl | 49.5 | 77.9 |
| Prochloraz | 60.3 | 72.0 |
| Dicofol | 19.0 | 30.6 |

As shown in Table1, the enzyme CYP2B10 shows inhibition by the tested compounds in the tested conditions, showing a strong signal specially for chlorpyrifos-ethyl, chlorpyrifos-methyl and prochloraz at the lower concentration tested.

### Example 2 - Effect of dimethoate in CYP2B11 assay

This assay was performed in a 96-well plate by adding 50 µL of 2 mg/L dimethoate chemical standard to wells in duplicate, previously prepared in assay buffer with 5% acetonitrile. Enzyme mixture consisted of 10 pmol/mL CYP2B11 (Cypex Limited) in the assay buffer: 75 mM potassium phosphate supplemented with 5 mM MgCl₂, pH=8.0, containing 333 mM Glucose-6-phosphate and 30 U/mL Glucose-6-phosphate dehydrogenase. After preparation, 50 µL of enzyme mixture were added to each plate and incubated for 10 minutes at 37°C. After incubation, 10 µL of a substrate mixture containing 30 µM NADP⁺ and 3 µM BOMCC in the phosphate buffer was added to the wells. The plate was incubated for 60 minutes at 37°C, and the fluorescence was monitored using 415/460 nm as excitation and emission wavelengths, respectively. A blank sample containing buffer solution was measured in same conditions and used to calculate the inhibition of 56.8% of the enzyme activity, that was associated with the presence of 2 mg/L of dimethoate pesticide, as shown in Figure 1. Considering the inhibition percentage determined, it is possible to confirm that CYP2B11 enzyme is susceptible to be inhibited by dimethoate pesticide.

### Example 3 - Effect of reporter substrate mix in CYP2B10 inhibition assay - dicofol pesticide determination

The enzyme CYP2B10 was exposed to 400 µg/L of dicofol pesticide, and the inhibition percentage was registered when reporter substrate concentration and composition was changed.

This assay was performed in a 96-well plate by adding 50 µL of chemical standard of pesticide in duplicate, previously prepared in assay buffer with 5% acetonitrile. Enzyme mixture consisted of 15 pmol/mL CYP2B10 (Cypex Limited) in the assay buffer: 75 mM potassium phosphate supplemented with 5 mM MgCl₂, pH=8.0, containing 333 mM Glucose-6-phosphate and 30 U/mL Glucose-6-phosphate dehydrogenase. After preparation, 50 µL of enzyme mixture were added to each plate and incubated for 10 minutes at 37°C. After incubation, 10 µL of a substrate mixture containing 30 µM NADP⁺ and different ratios of MOBFC and MOFC (total concentration, 10 µM) in the phosphate buffer (Table 2), were added to the wells. The plate was incubated for 60 minutes at 37°C, and the fluorescence was monitored using 370/520 nm as excitation and emission wavelengths, respectively. A blank sample containing buffer solution was measured in same conditions and used to calculate the inhibition of the enzyme activity that was associated with the presence of 400 µg/L dicofol.

**Table 2. Inhibition percentage (I (%)) calculated for CYP2B10 reactions in presence of 400 µg/L of dicofol and different mixtures of reporter substrates.**

| **MOBFC (µM)** | **MOFC (µM)** | **I (%)** |
|---|---|---|
| 10.0 | 0.0 | 16.8 |
| 5.0 | 5.0 | 24.3 |
| 3.3 | 6.6 | 37.4 |
| 2.0 | 8.0 | 50.6 |
| 0.0 | 10.0 | 72.9 |

As shown in Table 2, the reporter substrate is an important factor to define the method sensitivity to pesticides. The use of different proportions of reporter substrates affected drastically the inhibitory effect of the dicofol pesticide concentration tested on the CYP2B10 assay.

### Example 4 - Pesticides screening using CYP2B11 inhibition assay

An inhibition assay was performed using the enzyme CYP2B11 by contacting it to 400 µg/L of piperonyl butoxide, dicofol, diazinon, prochloraz, chlorpyrifos-methyl, chlorpyrifos-ethyl, iprodione, procymidone, DDT, penconazole, quinoxyfen, diuron, cypermethrin, deltamethrin and chlorothalonil.

This assay was performed in a 96-well plate by adding 50 µL of chemical standard of pesticides in duplicate, previously prepared in assay buffer with 5% acetonitrile. Enzyme mixture consisted of 5 pmol/mL CYP2B11 (Cypex Limited) in the assay buffer: 75 mM potassium phosphate supplemented with 5 mM MgCl₂, pH=8.0, containing 333 mM Glucose-6-phosphate and 30 U/mL Glucose-6-phosphate dehydrogenase. After preparation, 50 µL of enzyme mixture were added to each plate and incubated for 10 minutes at 37°C. After incubation, 10 µL of a substrate mixture containing 30 µM NADP⁺ and 3 µM BOMCC in the phosphate buffer was added to the wells. The plate was incubated for 60 minutes at 37°C, and the fluorescence was monitored using 410/460 nm as excitation and emission wavelengths, respectively. A blank sample containing buffer solution was measured in same conditions and used to calculate the inhibition of the enzyme activity that was associated with the presence of pesticides concentration.

**Table 3. Inhibition percentage (I (%)) calculated for CYP2B11 reactions in presence 400 µg/L of different pesticides.**

| **Pesticide** | **I(%)** |
|---|---|
| Chlorothalonil | 62.3 |
| Chlorpyrifos-ethyl | 94.2 |
| Chlorpyrifos-methyl | 96.0 |
| Cypermethrin | 17.5 |
| DDT | 70.7 |
| Deltamethrin | 51.0 |
| Diazinon | 84.1 |
| Dicofol | 79.0 |
| Diuron | 19.9 |
| Iprodione | 15.9 |
| Penconazole | 87.8 |
| Piperonyl butoxide | 65.6 |
| Prochloraz | 94.6 |
| Procymidone | 24.5 |
| Quinoxyfen | 18.1 |

As shown in table 3, the enzyme CYP2B11 shows considerable inhibition percentage values for the tested compounds at 400 µg/L. This enzyme can be used for the determination of a wide range of pesticide molecules.

### Example 5 - Effect of metrafenone pesticide in CYP2B11 and human homologous CYP2B6 activities

This assay was performed in a 96-well plate by adding 50 µL of 500 µg/L of metrafenone chemical standard to wells in duplicate, previously prepared in assay buffer with 5% acetonitrile. CYP2B11 enzyme mixture consisted of 5 pmol/mL Dog CYP2B11 bactosomes (Cypex Limited) in the assay buffer: 75 mM potassium phosphate supplemented with 5 mM MgCl₂, pH=8.0, containing 333 mM Glucose-6-phosphate and 30 U/mL Glucose-6-phosphate dehydrogenase. CYP2B6 enzyme mixture consisted of 2.5 pmol/mL of Vivid^{®} Human CYP2B6 baculosomes (Thermofisher Scientific) in the assay buffer: 75 mM potassium phosphate, pH=8.0, containing 333 mM Glucose-6-phosphate and 30 U/mL Glucose-6-phosphate dehydrogenase.

After preparation, 50 µL of each enzyme mixture were added to each plate and incubated for 10 minutes at 37°C. After incubation, 10 µL of a substrate mixture containing 30 µM NADP⁺ and 3 µM BOMCC in the phosphate buffer was added to the wells. The plate was incubated for 60 minutes at 37°C, and the fluorescence was monitored using 410/460 nm as excitation and emission wavelengths, respectively. A blank sample containing buffer solution was measured in same conditions and used to calculate the inhibition percentage of the activity of the enzymes in presence of the pesticide metrafenone.

**Table 4. Inhibition percentage (I (%)) calculated for human CYP2B6 and CYP2B11 reactions in presence 500 µg/L of metrafenone pesticide.**

| **CYP450** | **Enzyme concentration (pmol/mL)** | **BOMCC concentration (µM)** | **I (%)** |
|---|---|---|---|
| CYP2B6 | 2.5 | 3 | 0.0 |
| CYP2B11 | 5 | 3 | 35.2 |

The comparative metrafenone inhibition assay between CYP2B11 and human CYP2B6, shows that the enzyme CYP2B6 is not sensitive to metrafenone and the enzyme CYP2B11 presents a selective inhibition, being preferable its use for the detection of metrafenone on samples.

### Example 6 - Matrix effect of human CYP2B6 and mouse CYP2B10 in cultivated fungi extracts (mushrooms)

The present invention was applied to a blank extract (without added pesticides) of cultivated fungi samples. Briefly, the extraction method of the mushroom sample consisted of an acid treatment using a water:acetonitrile solution with 2% acetic acid, followed by the addition of sodium acetate to digest 10 g of minced mushroom. After centrifugation, the supernatant is diluted to 55% in water, and a clean-up step is performed using C18 cartridge. 2 mL of acetonitrile:water solution (75:25, v/v) with 0.1% acetic acid is used to elute the corresponding matrix fraction. The eluted extract was submitted to a freeze-out step at -20ºC during 20 min. The resulting supernatant (matrix fraction) is eluted through a cartridge containing NH₂ resin and neutral alumina, after which is evaporated and resuspended in the assay working buffer containing 10% acetonitrile.

This assay was performed in a 96-well plate by adding 50 µL of matrix fraction to wells in duplicate, previously prepared in assay buffer. CYP2B10 enzyme mixture consisted of 2 pmol/mL Mouse CYP2B10 bactosomes (Cypex Limited) in the assay buffer: 75 mM potassium phosphate supplemented with 5 mM MgCl₂, pH=8.0, containing 333 mM Glucose-6-phosphate and 30 U/mL Glucose-6-phosphate dehydrogenase. CYP2B6 enzyme mixture consisted of 2.5 pmol/mL of Vivid^{®} Human CYP2B6 baculosomes (Thermofisher Scientific) in the assay buffer: 75 mM potassium phosphate, pH=8.0, containing 333 mM Glucose-6-phosphate and 30 U/mL Glucose-6-phosphate dehydrogenase.

After preparation, 50 µL of each enzyme mixture were added to each plate and incubated for 10 minutes at 37°C. After incubation, 10 µL of a substrate mixture containing 30 µM NADP⁺ and 10 µM MOBFC in the phosphate buffer was added to the wells. The plate was incubated for 60 minutes at 37°C, and the fluorescence was monitored using 370/520 nm as excitation and emission wavelengths, respectively. A blank sample containing buffer solution was measured in same conditions and used to calculate the inhibition percentage (I (%)) of the extract and estimate the matrix effect expected for pesticides determination in mushrooms.

**Table 5. Matrix effect of mushrooms extracts estimated by the inhibition percentage (I (%)) for human CYP2B6 and mouse CYP2B10.**

| **CYP450** | **I (%)** |
|---|---|
| Human CYP2B6 | 16.1 |
| Mouse CYP2B10 | 1.0 |

As shown in Table 5, human CYP2B6 is more prone to the matrix effect in mushroom extract. Therefore, mouse CYP2B10 is preferable to perform the determination of pesticides in mushroom extracts since the matrix effect is negligible.

### Example 7 - Detection of prochloraz and chlorpyrifos-methyl in cultivated fungi extracts (mushrooms)

The present invention was applied to the detection of prochloraz and chlorpyrifos-methyl in an extract of cultivated fungi samples. Briefly, the extraction method of the mushroom sample consisted of an acid treatment using a water:acetonitrile solution with 2% acetic acid, followed by the addition of sodium acetate to digest 10 g of minced mushroom. After centrifugation, the supernatant is diluted to 55% in water, and a clean-up step is performed using C18 cartridge. 2 mL of acetonitrile:water solution (75:25, v/v) with 0.1% acetic acid is used to elute the target pesticides. The eluted extract was submitted to a freeze-out step at -20ºC during 20 min. The resulting supernatant is eluted through a cartridge containing NH₂ resin and neutral alumina, after which is evaporated and resuspended in the assay working buffer containing 10% acetonitrile.

The bioassay was performed in 500 µL tubes and in triplicate. 50 µL of extract was added to the tubes. Enzyme mixture was prepared following the protocols provided by the manufacturer (Cypex Limited) and consisted of 15 pmol/mL of CYP2B10 Bactosomes in the assay buffer: 75 mM potassium phosphate supplemented with 5 mM MgCl₂, pH=8.0, containing 333 mM Glucose-6-phosphate and 30 U/mL Glucose-6-phosphate dehydrogenase. After the preparation, 50 µL of enzyme mixture were added to each tube. After reach room temperature 10 µL of substrate mixture (10 µM MOBFC and 30 µM NADP⁺ diluted in potassium phosphate buffer 50 mM) was added to each tube. The tubes were incubated 60 minutes at 37°C and when time passed, fluorescence signal was measured with a fluorometric reader, 370 nm and 520 nm were the excitation and emission wavelengths, respectively. A blank sample, buffer solution, was measured at the same time and the reduction of the signal, inhibition of enzyme activity, was calculated and associated with the presence of pesticide molecules.

This protocol was validated for 0.01 mg/kg of prochloraz and 0.01 mg/kg of chlorpyrifos-methyl, showing a significant difference against blank signal (mushrooms without added pesticides). The validation was carried out during seven days, comparing the signal obtained for triplicate samples of blank and doped mushroom.

**Table 6. Validation results for the use of CYP2B10 assay to determine prochloraz and chlorpyrifos-methyl in mushrooms.**

| Sample | Average I (%) | Standard deviation |
|---|---|---|
| Blank Mushroom | 24.8 | 10.1 |
| Prochloraz spiked mushroom | 54.2 | 14.3 |
| Chlorpyrifos-methyl spiked mushroom | 46.8 | 5.6 |

As shown in Table 6, the enzyme CYP2B10 is capable to distinguish a blank sample from the ones containing prochloraz and chlorpyrifos-methyl pesticides at 0.01 mg/Kg.

The EU regulation establishes the maximum residues levels (MRLs) of 3 mg/kg for prochloraz (Reg. (EU) 2020/192) and 0.01 mg/kg for chlorpyrifos-methyl (Reg. (EU) 2020/1085) in cultivated fungi, demonstrating that the method can be used for this propose.

### Example 8 - CYP2B homologues relative inhibition

CYP2B10 and CYP2B11 were inhibited respectively, by a wide range of pesticides at 400 µg/L, as shown in Table 7, demonstrating that the approach can be used for pesticide detection.

This assay was performed in a 96-well plate by adding 50 µL of 400 µg/L of pesticides chemical standards to wells in duplicate, previously prepared in assay buffer with 5% acetonitrile. CYP2B11 enzyme mixture consisted of 5 pmol/mL Dog CYP2B11 bactosomes (Cypex Limited) in the assay buffer: 75 mM potassium phosphate supplemented with 5 mM MgCl₂, pH=8.0, containing 333 mM Glucose-6-phosphate and 30 U/mL Glucose-6-phosphate dehydrogenase. CYP2B10 enzyme mixture consisted of 2 pmol/mL Mouse CYP2B10 bactosomes (Cypex Limited) in the assay buffer: 75 mM potassium phosphate supplemented with 5 mM MgCl₂, pH=8.0, containing 333 mM Glucose-6-phosphate and 30 U/mL Glucose-6-phosphate dehydrogenase. CYP2B6 enzyme mixture consisted of 2.5 pmol/mL of Vivid^{®} Human CYP2B6 baculosomes (Thermofisher Scientific) in the assay buffer: 75 mM potassium phosphate, pH=8.0, containing 333 mM Glucose-6-phosphate and 30 U/mL Glucose-6-phosphate dehydrogenase.

After preparation, 50 µL of each enzyme mixture were added to each plate and incubated for 10 minutes at 37°C. After incubation, 10 µL of a substrate mixture containing 30 µM NADP⁺ and 3 µM BOMCC were added to CYP2B11 wells, and 10 µL of a substrate mixture containing 30 µM NADP⁺ and 10 µM MOBFC were added to the CYP2B10 and CYP2B6 wells, both in the phosphate buffer. The plate was incubated for 60 minutes at 37°C, and the fluorescence was monitored using 410/460 nm and 370/520 nm as excitation and emission wavelengths, respectively. A blank sample containing buffer solution was measured in same conditions and used to calculate the inhibition percentage of the activity of the enzymes in presence of the pesticide

The value 100 represents the normalized values against the maximum inhibition percentage found among the CYP2B10, CYP2B11 and CYP2B6.

**Table 7. Inhibition values are relative values, i.e. for each pesticide, a value of 100 has been given for the enzyme showing the most inhibition, and 0 for the enzyme showing no inhibition.**

| Group/Family | Active substance | 2B10 mouse | 2B11 | 2B6 human |
|---|---|---|---|---|
| Azole | Myclobutanil | 40 | 100 | 0 |
| Azole | Propiconazole | 100 | 100 | 50 |
| Azole | Fenbuconazole | 80 | 100 | 60 |
| Azole | Penconazole | 100 | 100 | 80 |
| Azole | Prochloraz | 100 | 80 | 100 |
| Organochlorine | Dicofol | 50 | 100 | 25 |
| Organophosphate | Chlorpyrifos | 50 | 80 | 100 |
| Organophosphate | Chlorpyrifos methyl | 70 | 80 | 100 |
| Organophosphate | Diazinon | 40 | 80 | 100 |
| Organophosphate | Fenitrothion | 100 | 100 | 100 |
| Organophosphate | Pirimiphos methyl | 60 | 100 | 100 |
| Phthalimide | Captan | 60 | 60 | 100 |
| Phthalimide | Folpet | 50 | 100 | 100 |
| Phthalimide/organophosphate | Phosmet | 80 | 100 | 80 |
| Triazine | Simazine | 100 | 100 | 0 |

Furthermore, CYP2B10 and CYP2B11, show selective inhibition and/or higher sensitivity to certain pesticide molecules, not observed with the human homologue (CYP2B6). Moreover, as shown in previous Example 6, CYP2B10 exhibits lower matrix effect for the determination of pesticides in mushroom samples, being preferable to be used over the CYP2B6, despite the lower inhibition percentage and sensitivity found for several of the tested pesticide molecules.

## Claims

1. Method for pesticide detection in a sample, comprising the following steps:
a) contacting the sample or an extract thereof, with, at least, one CYP450 enzyme selected from the list consisting of CYP2B11 enzyme and CYP2B10 enzyme, preferably with a CYP2B11 enzyme and a CYP2B10 enzyme;
b) addition of, at least, one enzyme reporter substrate;
c) detection and measurement of a detectable signal; and
d) determination of pesticide presence in the sample by comparing the measured detectable signal with a reference value.

2. Method according to claim 1, wherein the step (a) further comprises contacting the sample with, at least, one enzyme selected from the list consisting of acid phosphatase enzyme, acetylcholinesterase enzyme, aldehyde dehydrogenase enzyme, alkaline phosphatase enzyme, glutathione S-transferase enzyme, laccase enzyme, CYP450 enzyme, methyl parathion hydrolase enzyme, organophosphorus hydrolase enzyme, peroxidase enzyme, tyrosinase enzyme, urease enzyme, and any combination thereof.

3. Method according to claim 1 or 2, wherein the sample is selected from the list consisting of, food sample, feed sample, environmental sample, and an isolated biological sample.

4. Method according to any one of claims 1 to 3, wherein pesticide detection comprises the detection of, at least, one pesticide wherein the pesticide belongs to a family of pesticides selected from the list consisting of amide, aryl phenyl ketone, azole, benzophenone, dicarboximide, dinitrophenol, dithiocarbamate, organobromide, organochlorine, organophosphate, phthalimide, pyrethroid, triazine, synergists molecules and any combination thereof.

5. Method according to any one of claims 1 to 4, wherein the pesticide detection comprises the detection of, at least, one pesticide selected from the list consisting of benalaxyl, captan, chlorpyrifos, chlorpyrifos methyl, chlorothalonil, cyazofamid, cypermethrin, DDT, deltamethrin, diazinon, dicofol, dimethoate, epoxiconazole, fenbuconazole, fenitrothion, folpet, mancozeb, meptyldinocap, metrafenone, myclobutanil, penconazole, phosmet, piperonyl butoxide, pirimiphos methyl, prochloraz, procymidone, propiconazole, simazine and any combination thereof.

6. Kit for pesticide detection, comprising a CYP2B11 enzyme and a CYP2B10 enzyme, and at least one enzyme reporter substrate.

7. Kit according to claim 6, which further comprises, at least, one enzyme selected from the list consisting of acid phosphatase enzyme, acetylcholinesterase enzyme, aldehyde dehydrogenase enzyme, alkaline phosphatase enzyme, glutathione S-transferase enzyme, laccase enzyme, CYP450 enzyme, methyl parathion hydrolase enzyme, organophosphorus hydrolase enzyme, peroxidase enzyme, tyrosinase enzyme, urease enzyme, and any combination thereof.

8. Use of the kit according to claim 6 or 7, in a method according to any one of claims 1 to 5.

9. Use of the kit according to claim 6 or 7, for pesticide detection in a sample.

10. Use according to claim 9, wherein the sample is selected from the list consisting of food sample, feed sample, environmental sample, and an isolated biological sample.

11. Use according to claim 9 or 10, wherein pesticide detection comprises the detection of, at least, one pesticide, wherein the pesticide belongs to a family of pesticides selected from the list consisting of amide, aryl phenyl ketone, azole, benzophenone, dicarboximide, dinitrophenol, dithiocarbamate, organobromide, organochlorine, organophosphate, phthalimide, pyrethroid, triazine, synergists molecules and any combination thereof.

12. Use of, at least, one CYP450 enzyme selected from the list consisting of CYP2B11 enzyme and CYP2B10 enzyme, preferably a CYP2B11 enzyme and a CYP2B10 enzyme, for pesticide detection in a sample.

13. Use according to claim 12, which further comprises the use of, at least, one enzyme selected from the list consisting of acid phosphatase enzyme, acetylcholinesterase enzyme, aldehyde dehydrogenase enzyme, alkaline phosphatase enzyme, glutathione S-transferase enzyme, laccase enzyme, CYP450 enzyme, methyl parathion hydrolase enzyme, organophosphorus hydrolase enzyme, peroxidase enzyme, tyrosinase enzyme urease enzyme, and any combination thereof.

14. Use according to claim 12 or 13, wherein the sample is selected from the list consisting of, food sample, feed sample, environmental sample, and an isolated biological sample.

15. Use according to any one of claims 12 to 14, wherein pesticide detection comprises the detection of, at least, one pesticide wherein the pesticide belongs to a family of pesticides selected from the list consisting of amide, aryl phenyl ketone, azole, benzophenone, dicarboximide, dinitrophenol, dithiocarbamate, organobromide, organochlorine, organophosphate, phthalimide, pyrethroid, triazine, synergists molecules and any combination thereof.

## Patentansprüche

1. Verfahren zum Nachweis von Pestiziden in einer Probe, umfassend die folgenden Schritte:
a) Inkontaktbringen der Probe oder eines Extrakts davon mit mindestens einem CYP450-Enzym, das aus der Liste ausgewählt ist, die aus dem CYP2B11-Enzym und dem CYP2B10-Enzym besteht, vorzugsweise mit einem CYP2B11-Enzym und einem CYP2B10-Enzym;
b) Zugabe von mindestens einem Enzym-Reportersubstrat;
c) Nachweis und Messung eines nachweisbaren Signals; und
d) Bestimmung des Vorhandenseins von Pestiziden in der Probe durch Vergleich des gemessenen nachweisbaren Signals mit einem Referenzwert.

2. Verfahren nach Anspruch 1, wobei der Schritt (a) ferner das Inkontaktbringen der Probe mit mindestens einem Enzym umfasst, das aus der Liste ausgewählt ist, die aus dem Enzym saure Phosphatase, dem Enzym Acetylcholinesterase, dem Enzym Aldehyddehydrogenase, dem Enzym alkalische Phosphatase, dem Enzym Glutathion-S-Transferase, dem Enzym Laccase, dem Enzym CYP450, dem Enzym Methylparathionhydrolase, dem Enzym Organophosphorhydrolase, dem Enzym Peroxidase, dem Enzym Tyrosinase, dem Enzym Urease und einer beliebigen Kombination davon besteht.

3. Verfahren nach Anspruch 1 oder 2, wobei die Probe aus der Liste ausgewählt wird, die aus einer Lebensmittelprobe, einer Futtermittelprobe, einer Umweltprobe und einer isolierten biologischen Probe besteht.

4. Verfahren nach einem der Ansprüche 1 bis 3, wobei der Nachweis von Pestiziden den Nachweis von mindestens einem Pestizid umfasst, wobei das Pestizid zu einer Familie von Pestiziden gehört, die aus der Liste ausgewählt ist, die aus Amid, Arylphenylketon, Azol, Benzophenon, Dicarboximid, Dinitrophenol, Dithiocarbamat, Organobromid, Organochlor, Organophosphat, Phthalimid, Pyrethroid, Triazin, Synergistmolekülen und jeder Kombination davon besteht.

5. Verfahren nach einem der Ansprüche 1 bis 4, wobei der Pestizidnachweis den Nachweis von mindestens einem Pestizid umfasst, das aus der Liste ausgewählt ist, die aus Benalaxyl, Captan, Chlorpyrifos, Chlorpyrifos-Methyl, Chlorothalonil, Cyazofamid, Cypermethrin, DDT, Deltamethrin, Diazinon, Dicofol, Dimethoat, Epoxiconazol, Fenbuconazol, Fenitrothion, Folpet, Mancozeb, Meptyldinocap, Metrafenon, Myclobutanil, Penconazol, Phosmet, Piperonylbutoxid, Pirimiphos-Methyl, Prochloraz, Procymidon, Propiconazol, Simazin und jeder Kombination davon besteht.

6. Kit zum Nachweis von Pestiziden, umfassend ein CYP2B11-Enzym und ein CYP2B10-Enzym sowie mindestens ein Enzym-Reportersubstrat.

7. Kit nach Anspruch 6, das ferner mindestens ein Enzym umfasst, das aus der Liste ausgewählt ist, die aus dem Enzym saure Phosphatase, dem Enzym Acetylcholinesterase, dem Enzym Aldehyddehydrogenase, dem Enzym alkalische Phosphatase, dem Enzym Glutathion-S-Transferase, dem Enzym Laccase, dem Enzym CYP450, dem Enzym Methylparathionhydrolase, dem Enzym Organophosphorhydrolase, dem Enzym Peroxidase, dem Enzym Tyrosinase, dem Enzym Urease und jeder Kombination davon besteht.

8. Verwendung des Kits nach Anspruch 6 oder 7 in einem Verfahren nach einem der Ansprüche 1 bis 5.

9. Verwendung des Kits nach Anspruch 6 oder 7 zum Nachweis von Pestiziden in einer Probe.

10. Verwendung nach Anspruch 9, wobei die Probe aus der Liste ausgewählt ist, die aus einer Lebensmittelprobe, einer Futtermittelprobe, einer Umweltprobe und einer isolierten biologischen Probe besteht.

11. Verwendung nach Anspruch 9 oder 10, wobei der Nachweis von Pestiziden den Nachweis von mindestens einem Pestizid umfasst, wobei das Pestizid zu einer Familie von Pestiziden gehört, die aus der Liste ausgewählt ist, die aus Amid, Arylphenylketon, Azol, Benzophenon, Dicarboximid, Dinitrophenol, Dithiocarbamat, Organobromid, Organochlor, Organophosphat, Phthalimid, Pyrethroid, Triazin, Synergistmolekülen und jeder Kombination davon besteht.

12. Verwendung von mindestens einem CYP450-Enzym, das aus der Liste ausgewählt ist, die aus dem CYP2B11-Enzym und dem CYP2B10-Enzym besteht, vorzugsweise einem CYP2B11-Enzym und einem CYP2B10-Enzym, zum Nachweis von Pestiziden in einer Probe.

13. Verwendung nach Anspruch 12, die ferner die Verwendung von mindestens einem Enzym umfasst, das aus der Liste ausgewählt ist, die aus dem Enzym saure Phosphatase, dem Enzym Acetylcholinesterase, dem Enzym Aldehyddehydrogenase, dem Enzym alkalische Phosphatase, dem Enzym Glutathion-S-Transferase, dem Enzym Laccase, dem Enzym CYP450, dem Enzym Methylparathionhydrolase, dem Enzym Organophosphorhydrolase, dem Enzym Peroxidase, dem Enzym Tyrosinase, dem Enzym Urease und jeder Kombination davon besteht.

14. Verwendung nach Anspruch 12 oder 13, wobei die Probe aus der Liste ausgewählt ist, die aus einer Lebensmittelprobe, einer Futtermittelprobe, einer Umweltprobe und einer isolierten biologischen Probe besteht.

15. Verwendung nach einem der Ansprüche 12 bis 14, wobei der Pestizidnachweis den Nachweis von mindestens einem Pestizid umfasst, wobei das Pestizid zu einer Familie von Pestiziden gehört, die aus der Liste ausgewählt ist, die aus Amid, Arylphenylketon, Azol, Benzophenon, Dicarboximid, Dinitrophenol, Dithiocarbamat, Organobromid, Organochlor, Organophosphat, Phthalimid, Pyrethroid, Triazin, Synergistmolekülen und jeder Kombination davon besteht.

## Revendications

1. Procédé de détection de pesticides dans un échantillon, comprenant les étapes suivantes :
a) mise en contact de l'échantillon ou d'un extrait de celui-ci avec au moins une enzyme CYP450 choisie dans la liste constituée par l'enzyme CYP2B11 et l'enzyme CYP2B10, de préférence avec une enzyme CYP2B11 et une enzyme CYP2B10 ;
b) ajout d'au moins un substrat rapporteur d'enzyme ;
c) détection et mesure d'un signal détectable ; et
d) détermination de la présence de pesticides dans l'échantillon en comparant le signal détectable mesuré à une valeur de référence.

2. Procédé selon la revendication 1, dans lequel l'étape (a) comprend en outre la mise en contact de l'échantillon avec au moins une enzyme choisie dans la liste constituée par l'enzyme phosphatase acide, l'enzyme acétylcholinestérase, l'enzyme aldéhyde déshydrogénase, l'enzyme phosphatase alcaline, l'enzyme glutathion S-transférase, l'enzyme laccase, l'enzyme CYP450, l'enzyme méthylparathion hydrolase, l'enzyme organophosphorée hydrolase, l'enzyme peroxydase, l'enzyme tyrosinase, l'enzyme uréase, et toute combinaison de celles-ci.

3. Procédé selon la revendication 1 ou 2, dans lequel l'échantillon est choisi dans la liste constituée par un échantillon alimentaire, un échantillon d'aliment pour animaux, un échantillon environnemental, et un échantillon biologique isolé.

4. Procédé selon l'une quelconque des revendications 1 à 3, dans lequel la détection de pesticides comprend la détection d'au moins un pesticide dans lequel le pesticide appartient à une famille de pesticides choisie dans la liste constituée par un amide, une aryl phényl cétone, un azole, une benzophénone, un dicarboximide, un dinitrophénol, un dithiocarbamate, un organobromure, un organochlorine, un organophosphate, un phtalimide, un pyréthrinoïde, une triazine, des molécules synergistes et toute combinaison de ceux-ci.

5. Procédé selon l'une quelconque des revendications 1 à 4, dans lequel la détection de pesticides comprend la détection d'au moins un pesticide choisi dans la liste constituée par le bénalaxyle, le captane, le chlorpyrifos, le chlorpyrifos-méthyl, le chlorothalonil, le cyazofamide, la cyperméthrine, le DDT, la deltaméthrine, le diazinon, le dicofol, le diméthoate, l'époxiconazole, le fenbuconazole, le fénitrothion, le folpel, le mancozèbe, le méptyldinocap, la métrafénone, le myclobutanil, le penconazole, le phosmet, le pipéronyl butoxyde, le pirimiphos-méthyl, le prochloraze, la procymidone, le propiconazole, la simazine et toute combinaison de ceux-ci.

6. Kit de détection de pesticides, comprenant une enzyme CYP2B11 et une enzyme CYP2B10, et au moins un substrat rapporteur d'enzyme.

7. Kit selon la revendication 6, qui comprend en outre au moins une enzyme choisie dans la liste constituée par l'enzyme phosphatase acide, l'enzyme acétylcholinestérase, l'enzyme aldéhyde déshydrogénase, l'enzyme phosphatase alcaline, l'enzyme glutathion S-transférase, l'enzyme laccase, l'enzyme CYP450, l'enzyme méthylparathion hydrolase, l'enzyme organophosphorée hydrolase, l'enzyme peroxydase, l'enzyme tyrosinase, l'enzyme uréase, et toute combinaison de celles-ci.

8. Utilisation du kit selon la revendication 6 ou 7, dans un procédé selon l'une quelconque des revendications 1 à 5.

9. Utilisation du kit selon la revendication 6 ou 7, pour la détection de pesticides dans un échantillon.

10. Utilisation selon la revendication 9, dans laquelle l'échantillon est choisi dans la liste constituée par un échantillon alimentaire, un échantillon d'aliment pour animaux, un échantillon environnemental, et un échantillon biologique isolé.

11. Utilisation selon la revendication 9 ou 10, dans lequel la détection de pesticides comprend la détection d'au moins un pesticide, dans lequel le pesticide appartient à une famille de pesticides choisie dans la liste constituée par un amide, une aryl phényl cétone, un azole, une benzophénone, un dicarboximide, un dinitrophénol, un dithiocarbamate, un organobromure, un organochlorine, un organophosphate, un phtalimide, un pyréthrinoïde, une triazine, des molécules synergistes et toute combinaison de ceux-ci.

12. Utilisation d'au moins une enzyme CYP450 choisie dans la liste constituée par l'enzyme CYP2B11 et l'enzyme CYP2B10, de préférence une enzyme CYP2B11 et une enzyme CYP2B10, pour la détection de pesticides dans un échantillon.

13. Utilisation selon la revendication 12, qui comprend en outre l'utilisation d'au moins une enzyme choisie dans la liste constituée par l'enzyme phosphatase acide, l'enzyme acétylcholinestérase, l'enzyme aldéhyde déshydrogénase, l'enzyme phosphatase alcaline, l'enzyme glutathion S-transférase, l'enzyme laccase, l'enzyme CYP450, l'enzyme méthylparathion hydrolase, l'enzyme organophosphorée hydrolase, l'enzyme peroxydase, l'enzyme tyrosinase, l'enzyme uréase, et toute combinaison de celles-ci.

14. Utilisation selon la revendication 12 ou 13, dans laquelle l'échantillon est choisi dans la liste constituée par un échantillon alimentaire, un échantillon d'aliment pour animaux, un échantillon environnemental, et un échantillon biologique isolé.

15. Utilisation selon l'une quelconque des revendications 12 à 14, dans lequel la détection de pesticides comprend la détection d'au moins un pesticide, dans lequel le pesticide appartient à une famille de pesticides choisie dans la liste constituée par un amide, une aryl phényl cétone, un azole, une benzophénone, un dicarboximide, un dinitrophénol, un dithiocarbamate, un organobromure, un organochlorine, un organophosphate, un phtalimide, un pyréthrinoïde, une triazine, des molécules synergistes et toute combinaison de ceux-ci.
